# EUROPEAN PATENT SPECIFICATION

(11) **EP 1 801 215 B1**
(45) Date of publication and mention of the grant of the patent: **27.02.2013**
(21) Application number: 06026160.9
(22) Date of filing: 08.05.2000
(51) Int. Cl.: C12N 15/11, C12N 15/63, C12N 15/82, A01N 63/00, A01H 5/00

(54) **Regulation of viral gene expression**
Regulierung der viralen Genexpression
Régulation de l'expression d'un gène viral

(30) Priority: 10.05.1999 US 309038
(43) Date of publication of application: 27.06.2007
(62) Divisional of application: 00927165.1
(73) Proprietor: Syngenta Participations AG, 4058 Basel (CH)
(72) Inventor: Heifetz, Peter Bernard, Durham, NC 27713 (US); Patton, David Andrew, Syngenta Biotechnology, Inc., Research Triangle Park, NC 27709 (US); Levin, Joshua Zvi, Durham, NC 27701 (US); Que, Qiudeng, c/o Syngenta Biotechnology, Inc., Research Triangle Park, NC 27709 (US); De Haan, Petrus Theodorus, 1602 GL Enkhuizen (NL); Gielen, Johannes Jacobus Ludgerus, 31790 Saint-Sauveur (FR)
(74) Representative: Bessiere, Philippe Jean Luc

(56) References cited:
- WO-A-00/44914
- WO-A-90/14090
- WO-A-98/36083
- WO-A-98/37223
- WO-A-98/53083
- WO-A-99/15682
- WO-A-99/32619
- WO-A-99/49029
- WO-A-99/53050
- WO-A-99/61631
- WO-A2-91/13159
- WATERHOUSE ET AL: "Virus resistance and gene silencing in plants can be induced by simultaneous expression of sense and antisense RNA" PROCEEDINGS OF THE NATIONAL ACADEMY OF SCIENCES OF USA,US,NATIONAL ACADEMY OF SCIENCE. WASHINGTON, vol. 95, no. 95, November 1998 (1998-11), pages 13959-13964, XP002114472 ISSN: 0027-8424
- SIJEN TITIA ET AL: "RNA-mediated virus resistance: Role of repeated transgenes and delineation of targeted regions." PLANT CELL, vol. 8, no. 12, 1996, pages 2277-2294, XP002146482 ISSN: 1040-4651
- BAULCOMBE D: "Mechanisms of pathogen-dereived resistance to viruses in transgenic plants" PLANT CELL,US,AMERICAN SOCIETY OF PLANT PHYSIOLOGISTS, ROCKVILLE, MD, vol. 8, no. 10, 1 October 1996 (1996-10-01), pages 1833-1844, XP002095878 ISSN: 1040-4651
- MONTGOMERY M K ET AL: "Double-stranded RNA as a mediator in sequence-specific genetic silencing and co-suppression" TRENDS IN GENETICS,NL,ELSEVIER SCIENCE PUBLISHERS B.V. AMSTERDAM, vol. 14, no. 7, 1 July 1998 (1998-07-01), pages 255-258, XP004124680 ISSN: 0168-9525
- GRANT SARAH R: "Dissecting the mechanisms of posttranscriptional gene silencing: Divide and conquer." CELL, vol. 96, no. 3, 5 February 1999 (1999-02-05), pages 303-306, XP002146483 ISSN: 0092-8674
- MANNERLOF ET AL: "reduced titer of BNYVV in transgenic sugar beets expressing the BNYVV coat protein", EUPHYTICA, KLUWER ACADEMIC PUBLISHERS, NL LNKD- DOI:10.1007/BF00027479, vol. 90, no. 3, 1 January 1996 (1996-01-01), pages 293-299, XP002112359, ISSN: 0014-2336

## Description

The present disclosure relates to methods of altering the expression of viral genes in cells, plants or animals using sense and antisense RNA fragments of said genes, and to cells, plants or animals with altered viral gene expression obtained using the methods of the present invention. The disclosure particularly relates to such cells, plants or animals resistant or tolerant to viruses.

An area of deep interest is resistance or tolerance to viruses. Viruses affect most living organisms. In crops, large proportions of the harvest may be lost due to virus infections. Farm animals are also often infected by viruses and must sometimes be slaughtered to prevent spreading of the disease leading to dramatic economic consequences. Companoin animals are also affected by viruses, and, finally, viruses infect humans causing a lot of suffering. Although treatments against viruses have been developed, they are very often either extremely expensive or of limited efficiency.
It is desirable to modify cells, plants or animals so that the expression of a particular viral gene is altered to create cells, plants or animals resistant or tolerant to said virus. Current methods to alter the expression of a gene usually rely upon techniques of sense or antisense suppression. Unfortunately, these methods are often variable and unpredictable
in their ability to alter gene expression, and in many cases a complete disruption of the particular gene activity is not achieved.
Manner of et al (Euphytica (1996) vol. 90, p 293-299) and WO 91/13159 A2 (1991) both disclose creation of forming resistant plants using parts of the viral genome as targets.
There is therefore a long-felt but unfulfilled need for novel methods and compositions allowing one to effectively and predictably alter the expression of a viral gene to obtain cells, plants or animals with resistance or tolerance to viruses.

The present disclosure relates to methods of altering the expression of a viral gene in cells, plants or animals using sense and antisense RNA fragments of the gene. Importantly, such sense and antisense RNA fragments are capable of forming a double-stranded RNA molecule. Particularly, the present disclosure relates to methods and compositions of conferring upon a cell, plant or animal resistance or tolerance to viruses. Preferably the disclosure relates to methods of conferring upon a plant resistance or tolerance to viruses.
The disclosure also preferably relates to plant cells obtained using such methods, to plants derived from such cells, to the progeny of such plants and to seeds derived from such plants. In such plant cells or plants, the alteration of the gene expression of a particular viral gene is more effective, selective and more predictable than the alteration of the gene expression of a particular viral gene obtained using current methods known in the art.

The disclosure therefore provides:
A method comprising introducing into a cell a plurality of sub-sequences, e.g. RNA fragments or DNA sequences, characterized in that at least two of the sub-sequences have sense and antisense sequences of viral RNAs and are capable of forming a double-stranded RNA molecule. Preferably, the method comprises introducing into a cell an RNA which consists of a plurality of sub-sequences characterized in that at least two of the sub-sequences have the sequences of viral RNAs. Preferably, the RNA contains at least one translational stop codon located upstream of the 3' terminal sub-sequence. In another preferred disclosure, the method comprises introducing into a cell a nucleotide sequence or DNA molecule encoding expression of said RNA.

The disclosure further provides:
A method comprising introducing into a cell a first DNA sequence capable of expressing in said cell a sense RNA fragment of a viral target gene, and a second DNA sequence capable of expressing in said cell an antisense RNA fragment of said target gene, wherein said sense RNA fragment and said antisense RNA fragment are capable of forming a double-stranded RNA molecule, wherein the expression of said viral target gene in said cell is altered. In a preferred disclosure, the target gene comprises a viral genome or a portion thereof and the cell is preferably resistant or tolerant to viruses. In a preferred disclosure, the virus is selected from the group consisting of tospoviruses, potyviruses, potexviruses, tobamoviruses, luteoviruses, cucumoviruses, bromoviruses, closteorviruses, tombusviruses and furoviruses. In another preferred disclosure, the DNA sequences comprises a nucleotide sequence derived from a viral coat protein gene, a viral nucleocapsid protein gene, a viral replicase gene, a movement protein gene or portions thereof. In a further preferred embodiment, a cell is a plant cell, such as a monocotyledonous or a dicotyledonous cell. In a preferred disclosure, the the DNA sequences are stably integrated in the genome of the plant cell. In a preferred disclosure, the DNA molecule further comprises a promoter operably linked to said first or said second DNA sequence. In another preferred disclosure the first DNA sequence and the second DNA sequence are comprised in two different, i.e. separate DNA molecules.

Alternatively, the first DNA sequence and the second DNA sequence are comprised in one DNA molecule. In this case, the first DNA sequence and the second DNA sequence are preferably comprised in the same DNA strand of said DNA molecule meaning that the sense RNA fragment and the antisense RNA fragment are comprised in one RNA molecule. Preferably, the RNA molecule is capable of folding such that said RNA fragments comprised therein form a double-stranded region. Examples of such RNA molecules comprise the inverted repeat sequence of SEQ ID NO: 17 or SEQ ID NO: 18, The sense RNA fragment and the antisense RNA fragment can be comprised in or expressed as two RNA molecules. In this case, the first DNA sequence and the second DNA sequence are preferably operably linked to a bi-directional promoter or, alternatively, the first DNA sequence is operably linked to a first promoter and the second DNA sequence is operably linked to a second promoter, wherein the first promoter and the second promoter are the same promoter or different promoters. Preferably in the first DNA sequence and the second DNA sequence are comprised in complimentary strands of said DNA molecule.

In yet another preferred disclosure, the first DNA sequence is the complementary DNA strand of the second DNA sequence in said DNA molecule. In this case, the DNA molecule further comprises a first promoter operably linked to said first or second DNA sequence. In a preferred disclosure, the DNA molecule further comprises a first site-specific recombination site between said first promoter and said first or second DNA sequence and a second site-specific recombination site at the 3'-end of said first DNA sequence, wherein said first and second site-specific recombination sites are capable of inverting said first or second DNA sequence between said first and second site-specific recombination sites in presence of a site-specific recombinase. In a further preferred disclosure and as a result of said inverting said first promoter is capable of expressing said second (or first, depending on which DNA sequence was originally linked to the promoter) DNA sequence. The plant cell preferably further comprises a site-specific recombinase capable of recognizing said site-specific recombination sites.

Preferably the DNA molecule further comprises a first promoter operably linked to said first DNA sequence and a second promoter operably linked to said second DNA sequence, wherein the first promoter and the second promoter comprise the same promoter or comprise different promoters.

Preferably the promoter in the DNA molecule comprises a native promoter of said cell or the promoter is a heterologous promoter, for example a tissue specific promoter, a developmentally regulated promoter, a constitutive promoter or an inducible promoter. Optionally, the promoter is a divergent or bi-directional promoter capable of initiating transcription of DNA sequences on each side of the promoter.

The DNA sequence may further comprise a linker between the DNA sequences encoding the sense and antisense RNA fragments. The linker comprises, e.g. an expression cassette comprising a functional gene, e.g. a selectable marker gene or regulatory sequences, e.g. intron processing signals.

The disclosure also further provides:
A cell comprising the sense and antisense RNA fragments, wherein the expression of said viral target gene in said cell is altered by said RNA fragments. In a preferred disclosure, the cell is resistant or tolerant to viruses. Preferably the cell is a plant cell and the disclosure further provides a plant and the progeny thereof derived from the plant cell, and seeds derived from the plant.

The disclosure also provides:
DNA constructs comprising the DNA sequences of the present disclosure.

Such a DNA construct comprises a first DNA sequence capable of expressing in a cell a sense RNA fragment of a viral genome or portion thereof and a second DNA sequence capable of expressing in said cell an antisense RNA fragment of said viral genome or portion thereof, wherein said sense RNA fragment and said antisense RNA fragment are capable of forming a double-stranded RNA molecule.

The expression of said viral genome or portion thereof in said cell can be altered. In another preferred disclosure, the cell is a plant cell. In another preferred disclosure, the virus is selected from the group consisting of tospoviruses, potyviruses, potexviruses, tobamoviruses, luteoviruses, cucumoviruses, bromoviruses, closteorviruses, tombusviruses and furoviruses. The DNA sequences may comprise a nucleotide sequence derived from a viral coat protein gene, a viral nucleocapsid protein gene, a viral replicase gene, a movement protein gene or portions thereof. In yet another preferred disclosure, the DNA construct further comprises a promoter operably linked to said first or said second DNA sequence. In yet another preferred disclosure, the DNA construct comprises a first promoter operably linked to said first DNA sequence and a second promoter operably linked to said second DNA sequence.

The DNA construct may further comprise a bi-directional promoter operably linked to said first DNA sequence and said second DNA sequence.

The disclosure further provides:
A RNA construct comprising:
(a) a first DNA sequence capable of expressing in a cell a sense RNA fragment of a target gene and a second DNA sequence capable of expressing in said cell an antisense RNA fragment of said target gene, wherein said sense RNA fragment and said antisense RNA fragment are capable of forming a double-stranded RNA molecule, wherein said first DNA sequence is the complementary strands of said second DNA sequence in said DNA construct,
(b) a promoter operably linked to said first or second DNA sequence,
(c) a first site-specific recombination site between said promoter and said first or second DNA sequence, and
(d) a second site-specific recombination site at the 3'-end of said first or second DNA sequence, wherein said first and second site-specific recombination sites are capable of inverting said first or second DNA sequence between said first and second site-specific recombination sites in presence of a site-specific recombinase.

Preferably the expression of said target gene in said cell is altered.

The disclosure further provides:
A DNA construct comprising:
(a) a first DNA sequence capable of expressing in a cell a sense RNA fragment of a target gene and a second DNA sequence capable of expressing in said cell an antisense RNA fragment of said target gene, wherein said sense RNA fragment and said antisense RNA fragment are capable of forming a double-stranded RNA molecule, wherein said first DNA sequence is the complementary strands of said second DNA sequence in said DNA construct,
(b) a first promoter operably linked to said first DNA sequence,
(c) a second promoter operably linked to said second DNA sequence.

Preferably the expression of said target gene in said cell is altered.

A "double-stranded RNA (dsRNA)" molecule comprises a sense RNA fragment of a target gene and an antisense RNA fragment of the same target gene, which both comprise nucleotide sequences complementary to one another, thereby allowing the sense and antisense RNA fragments to pair and form a double-stranded RNA molecule.

"Complementary" refers to two nucleotide sequences which comprise antiparallel nucleotide sequences capable of pairing with one another upon formation of hydrogen bonds between the complementary base residues in the antiparallel nucleotide sequences.

"Antiparallel" refers herein to two nucleotide sequences paired through hydrogen bonds between complementary base residues with phosphodiester bonds running in the 5'-3' direction in one nucleotide sequence and in the 3'-5' direction in the other nucleotide sequence.

A "target gene" is any viral gene of known function or is a gene whose function is unknown, but whose total or partial nucleotide sequence is known. A target gene is an native gene of the cell or is a heterologous gene which had previously been introduced into the cell, preferably by genetic transformation or viral infection of the cell. Preferably, the target gene is a gene in a plant cell.

A "native" gene refers to a gene which is present in the genome of the untransformed cell.

An "essential" gene is a gene encoding a protein such as e.g. a biosynthetic enzyme, receptor, signal transduction protein, structural gene product, or transport protein that is essential to the growth or survival of the cell.

To "alter" the expression of a target gene in a cell means that the level of expression of the target gene in a cell after applying the method desenned herein different from its expression in the cell without applying the method. To alter gene expression preferably means that the expression of the target gene in the cell is reduced, preferably strongly reduced, more preferably the expression of the gene is not detectable, resulting in a knockout mutant phenotype in cells or plants or animals derived thereof.

"Isolated" as used herein, an isolated nucleic acid molecule that, by the hand of man, exists apart from its native environment and is therefore not a product of nature. An isolated nucleic acid molecule may exist in a purified form or may exist in a non-native environment such as, for example, a transgenic host cell.

"Expression cassette" as used herein means a DNA sequence capable of directing expression of a particular nucleotide sequence in an appropriate host cell, comprising a promoter operably linked to the nucleotide sequence of interest which is operably linked to termination signals. It also typically comprises sequences required for proper translation of the nucleotide sequence. The coding region usually codes for a protein of interest but may also code for a functional RNA of interest, for example antisense RNA or a nontranslated RNA, in the sense or antisense direction. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. Typically, however, the expression cassette is heterologous with respect to the host, i.e., the particular DNA sequence of the expression cassette does not occur naturally in the host cell and must have been introduced into the host cell or an ancestor of the host cell by a transformation event. The expression of the nucleotide sequence in the expression cassette may be under the control of a constitutive promoter or of an inducible promoter which initiates transcription only when the host cell is exposed to some particular external stimulus. In the case of a multicellular organism, such as a plant, the promoter can also be specific to a particular tissue or organ or stage of development.
"Heterologous" as used herein means "of different natural origin" or represents a non-natural state. For example, if a host cell is transformed with a nucleic acid sequence derived from another organism, particularly from another species, that nucleic acid sequence is heterologous with respect to that host cell and also with respect to descendants of the host cell which carry that nucleic acid sequence. Similarly, heterologous refers to a nucleotide sequence derived from and inserted into the same natural, original cell type, but which is present in a non-natural state, e.g. a different copy number, or under the control of different regulatory elements.

In its broadest sense, the term "substantially similar", when used herein with respect to a nucleotide sequence, means a nucleotide sequence corresponding to a reference nucleotide sequence, wherein the corresponding sequence encodes a polypeptide having substantially the same structure and function as the polypeptide encoded by the reference nucleotide sequence, e.g. where only changes in amino acids not affecting the polypeptide function occur. Desirably the substantially similar nucleotide sequence encodes the polypeptide encoded by the reference nucleotide sequence. The percentage of identity between the substantially similar nucleotide sequence and the reference nucleotide sequence (number of complementary bases in the complementary sequence divided by total number of bases in the complementary sequence) desirably is at least 80%, more desirably 85%, preferably at least 90%, more preferably at least 95%, still more preferably at least 99%.

"Regulatory elements" refer to sequences involved in conferring the expression of a nucleotide sequence. Regulatory elements comprise a promoter operably linked to the nucleotide sequence of interest and termination signals. They also typically encompass sequences required for proper translation of the nucleotide sequence.

A "plant" refers to any plant or part of a plant at any stage of development. Therein are also included cuttings, cell or tissue cultures and seeds. The term "plant tissue" includes, but is not limited to, whole plants, plant cells, plant organs, plant seeds, protoplasts, callus, cell cultures, and any groups of plant cells organized into structural and/or functional units.

"Virus resistance or tolerance" means herein that a resistant or tolerant cell, plant or animal is either not susceptible or has reduced susceptibility to one or more viruses as compared to a sensitive cell, plant or animal. Resistance or tolerance for instance means that the usual symptoms of a virus infection are absent or reduced, or that accumulation or replication of the virus in the cell is prevented or reduced, or that movement of the virus, e.g. from cell to cell is prevented or reduced.

By "alteration of the expression of the viral genome or of a portion thereof" is typically understood that accumulation, replication or movement of the virus or a portion or component thereof, e.g. a RNA, DNA or protein of the virus, in the cell is affected.

The present disclosure relates to methods to regulate, i.e. alter the expression of a viral gene in cells, plants or animals. Commonly available methods to regulate the expression of a gene in cells, plants or animals lock predictability and show variability depending upon which gene is to be regulated. The present method alleviates these problems and provides for reproducible and efficacious regulation of a gene in cells, plants or animals.

The gene the expression of which is regulated is a viral genome or a portion thereof. In a preferred disclosure, a cell is an eukaryotic cell, more preferably a plant cell, such as a monocotyledonous or a dicotyledonous cell, or an animal cell e.g. from a mammal, e.g. a human, a bovine, an ovine, a porcine, a feline or a canine, or from an avian.

The present disclosure utilizes a sense RNA fragment and an antisense RNA fragment of a viral target gene to alter the expression of the gene in a cell. In a first aspect the disclosure provides a method comprising introducing into a cell a sense RNA fragment of a target gene and an antisense RNA fragment of said target gene, wherein said sense RNA fragment and said antisense RNA fragment are capable of forming a double-stranded RNA molecule, wherein the expression of said target gene in said cell is altered. The RNA fragments are introduced in the cells by different transformation methods such as particle bombardment or PEG-mediated transformation or electroporation. In another preferred aspect, other techniques, such as microinjection of the RNA fragments, are used.

In a preferred aspect, the RNA fragments are comprised in two different RNA molecules. In this case, the RNA fragments are mixed before being introduced into said cell, e.g. under conditions allowing them to form a double-stranded RNA molecule. The RNA fragments can be introduced into said cell sequentially.

Preferably, the time interval between the introduction of each of the RNA molecules is short, preferably less than one hour. In yet another aspect, the RNA fragments are comprised in one RNA molecule. By using one single RNA molecule the two complementary RNA fragments are in close proximity such that pairing and is favored. In such case, the RNA molecule is preferably capable of folding such that said RNA fragments comprised therein form a double-stranded region. In this case, the complementary parts of the RNA fragments recognize one another, pair with each other and form the double-stranded RNA molecule. In a preferred aspect, the RNA fragments are incubated under conditions allowing them to form a double-stranded RNA molecule prior to introduction into the cell. In yet another aspect, the RNA molecule comprises a linker between the sense RNA fragment and the antisense RNA fragment. The linker preferably comprises a RNA sequence encoded by an expression cassette comprising a functional gene, e.g. a selectable marker gene. The linker may comprise a RNA sequence encoded by regulatory sequences, which e.g. comprise intron processing signals.

In a further aspect, the present disclosure provides a method of altering the expression of a viral genome comprising introducing into a cell a first DNA sequence capable of expressing in said cell a sense RNA fragment of said viral genome and a second DNA sequence capable of expressing in said cell an antisense RNA fragment of said viral genome, wherein said sense RNA fragment and said antisense RNA fragment are capable of forming a double-stranded RNA molecule. In a preferred disclosure the first DNA sequence and the second DNA sequence are stably integrated in the genome of the cell. In another preferred disclosure, the DNA sequences are comprised in two different DNA molecules. In another preferred disclosure the DNA sequences are comprised in one DNA molecule. In such case, the DNA molecule preferably encodes a single RNA molecule which comprises the sense and antisense RNA fragments. By using one single RNA molecule the two complementary RNA fragments are in close proximity such that pairing and is favored. The DNA molecule encodes two separate RNA molecules, e.g. one RNA molecule comprising a sense RNA fragment and one RNA molecule comprising an antisense RNA fragment. The single RNA molecule or the two distinct RNA molecules are preferably capable of folding such that said RNA fragments comprised therein form a double-stranded region, in which the complementary parts of the RNA fragments recognize one another, pair with each other and form the double-stranded RNA molecule.

The single DNA molecule or each of the two distinct DNA molecules may comprise a promoter operably linked to said DNA sequences.

The promoter in the DNA sequence may comprise a native plant promoter or the natural promoter of said viral gene to be inactivated, in order to insure that the double-stranded RNA is expressed in the same tissues and at the same time in development as the viral target gene. In another aspect the promoter is a heterologous promoter, for example a tissue specific promoter, a developmentally regulated promoter, a constitutive promoter or an inducible promoter.

In yet another aspect, the DNA sequence comprises a linker between the DNA sequences encoding said two complementary RNA fragments. The linker preferably comprises an expression cassette comprising a functional gene, e.g. a selectable marker gene. In another aspect, the linker comprises regulatory sequences, which e.g. comprise intron processing signals.

DNA molecules are transformed into cells using methods well-known in the art or described below. The present disclosure also provides a DNA construct comprising DNA sequences, a recombinant vector comprising such DNA constructs and a composition comprising DNA sequences .

In the present disclosure the complementary region between the sense and antisense RNA fragments is desirably at least 15 nucleotides long, more desirably at least 50 nucleotides long, and preferably at least 500 bp long. Preferably, the complementary region is less than 5 kb and more preferably less than 2 kb. In one particular aspect the complementary region between the sense and antisense RNA fragments comprises the coding region of the target gene. The complementary region may comprise untranslated regions (UTR) of the target gene, e.g. 5' UTR or 3' UTR. In yet another preferred aspect, a DNA sequence encoding a sense or antisense RNA fragment is derived from a c-DNA molecule or comprises regulatory elements of the viral target gene whose expression is to be altered, such as promoter or termination signals.

In another preferred aspect, the complementary region between the sense and antisense RNA fragments is identical to the corresponding sequence of the gene whose expression is altered. The complementary region between the sense and antisense RNA fragments can be substantially similar to the corresponding sequence of the gene whose expression is altered and is still capable of altering the expression of the gene. In this case, the complementary region is desirably at least 50% identical to the corresponding sequence of the gene whose expression is altered more desirably at least 70% identical, preferably at least 90% identical, more preferably at least 95% identical. Thereby, using a single double-stranded RNA molecule allows to alter the expression of a single gene or of a plurality of genes, the single gene comprising sequences identical to the double-stranded RNA or being substantially similar to the double-stranded RNA.

In another preferred aspect, the complementary region between the sense and antisense RNA fragments does not contain any mismatch between the sense and antisense RNA fragments. Preferably the complementary region between the sense and antisense RNA fragments comprises at least one mismatch between the sense and antisense RNA fragments, and the two RNA fragments are still capable of pairing and forming a double-stranded RNA molecule, thereby altering the expression of the gene. Desirably, there is less than 50% mismatch between the sense and antisense RNA fragments in the complementary region, more desirably less than 30% mismatch, preferably less than 20% mismatch, more preferably less than 10% mismatch, yet more preferably less than 5 % mismatch.

### Resistance or Tolerance to Viruses

The present disclosure results in cells, animals or plants which are virus resistant or tolerant. Viruses controlled using the present disclosure comprise but are not limited to dsDNA viruses, dsRNA viruses, plus-strand and minus-strand ssRNA viruses, ambisense RNA viruses and retroviruses. Preferably controlled are plant viruses, such astospoviruses (e.g. tomato spotted wilt virus abbreviated TSWV), potyviruses (e.g. turnip mosaic virus abbreviated TuMV, lettuce mosaic virus abbreviated LMV, water melon mosaic virus II abbreviated WMVII, Zucchini yellow mosaic virus abbreviated ZYMV, potato virus Y abbreviated PVY, and papaya ringspot virus abbreviated PRSV), potexviruses, tobamoviruses (e.g. pepper mild mottle virus abbreviated PMMV, and tomato mosaic virus abbreviate ToMV), luteoviruses (e.g. beet western yellows virus abbreviated BWYV, and beet mild yellowing virus abbreviated BMYV), cucumoviruses (e.g. cucumber mosaic virus abbreviated CMV), geminiviruses (e.g. tomato yellow leafcurl virus abbreviated TYLCV), caulimoviruses (e.g. Cauliflower mosaic virus abbreviated CaMV), bromoviruses, closteorviruses, tombusviruses and furoviruses (e.g. beet necrotic yellow vein virus abbreviated BNYVV. Additional classes of viruses which can be controlled are described in Zacomer et al. (1995) Journal of General Virology, 76: 231-247 and in Martelli (1992) Plant Disease, 76: 436-441. Preferred DNA sequences of a viral genome to achieve virus control corrspond to regions of the genes encoding viral coat proteins, viral nucleocapsid proteins, viral replicases, movement proteins and the like. Further nucleotide sequences useful to control virus genome expression are described in WO 95/09920. Preferred DNA sequences may include portions of the viral genome not translated into proteins, e.g. 5' or 3' untranslated regions.

Preferably, a method leads to resistance or tolerance to a broad-spectrum of viruses. For example, a method leads to resistance or tolerance to the virus encoded by the viral genome and other viruses in the same virus class, group or genus. Alternatively, a method leads to resistance or tolerance to the virus encoded by the viral genome and other isolates of the same virus. Also, a method leads to resistance or tolerance to the virus encoded by the viral genome and other viruses in the same virus group or genus in different species, preferably in related species, preferably species in which such viruses exist. Optionally, more than one pair, i.e. at least two pairs of sense and antisense RNA fragments which are capable of forming a dsRNA are used. Such pairs are for example derived from the same viral genome, but from different portions of the same viral genome. Alternatively, such pairs are derived from different viral genomes. Thus, resistance or tolerance to different virus classes, groups or genera is achieved using the present disclosure.

Plant cells and plants derived thereof, which are virus resistant or tolerant are preferably dicotyledonous plants. Methods to confer resistance or tolerance to furoviruses (see e.g. Rush and Heidel (1995) Plant Disease 79: 868-875) in sugar beet and canola are disclosed and described in further detail for BNYVV, the causal agent of rhizomania (crazy roots) in sugar-beet, in Example 9. Methods to confer resistance or tolerance to "virus yellow" (see e.g. CRC Handbook on Disease of Sugar Beet, Volume II, pp. 35-52) such as BMYV and beet western yellows virus (BWYV) that infect sugar beet and oilseed rape, respectively , are described in further detail in Example 8.

Methods also confer tolerance or resistance to viruses in monocotyledonous plants. For example, using the teachings of the present disclosure and of US patent 5,569,828 tolerance or resistance to Maize chlorotic dwarf virus is obtained. Similarly, using the present teachings and of US patent 5,428,144 tolerance or resistance to Maize dwarf mosaic virus is achieved.

### Plant Transformation Technology

DNA molecules are incorporated in plant or bacterial cells using conventional recombinant DNA technology. Generally, a DNA molecule is comprised in a transformation vector. A large number of such vector systems known in the art are used, such as plasmids, bacteriophage viruses and other modified viruses. The components of the expression system are also modified, e.g. to increase expression of the sense and antisense RNA fragments. For example, truncated sequences, nucleotide substitutions or other modifications are employed. Expression systems known in the art are used to transform virtually any crop plant cell under suitable conditions. A transgene comprising a DNA molecule is preferably stably transformed and integrated into the genome of the host cells. In another preferred aspect, the transgene comprising a DNA molecule is located on a self-replicating vector. Examples of self-replicating vectors are viruses, in particular gemini viruses. Transformed cells are preferably regenerated into whole plants.

Plants transformed in accordance with the present disclosure may be monocots or dicots and include, but are not limited to, maize, wheat, barley, rye, sweet potato, bean, pea, chicory, lettuce, cabbage, cauliflower, broccoli, turnip, radish, spinach, asparagus, onion, garlic, pepper, celery, squash, pumpkin, hemp, zucchini, apple, pear, quince, melon, plum, cherry, peach, nectarine, apricot, strawberry, grape, raspberry, blackberry, pineapple, avocado, papaya, mango, banana, soybean, tomato, sorghum, sugarcane, sugarbeet, sunflower, rapeseed, clover, tobacco, carrot, cotton, alfalfa, rice, potato, eggplant, cucumber, Arabidopsis, and woody plants such as coniferous and deciduous trees. Once a desired nucleotide sequence has been transformed into a particular plant species, it may be propagated in that species or moved into other varieties of the same species, particularly including commercial varieties, using traditional breeding techniques.

### A. Requirements for Construction of Plant Expression Cassettes

Gene sequences intended for expression in transgenic plants are first assembled in expression cassettes behind a suitable promoter expressible in plants. The expression cassettes may also comprise any further sequences required or selected for the expression of the transgene. Such sequences include e.g., but are not restricted to, transcription terminators, extraneous sequences to enhance expression such as introns, vital sequences, and sequences intended for the targeting of the gene product to specific organelles and cell compartments. These expression cassettes can then be easily transferred to the plant transformation vectors described *infra.* The following is a description of various components of typical expression cassettes.

### 1. Promoters

The selection of the promoter used in expression cassettes determine the spatial and temporal expression pattern of the transgene in the transgenic plant. Selected promoters express transgenes in specific cell types (such as leaf epidermal cells, mesophyll cells, root cortex cells) or in specific tissues or organs (roots, leaves or flowers, for example) and the selection reflects the desired location of accumulation of the gene product. Alternatively, the selected promoter drives expression of the gene under various inducing conditions. Promoters vary in their strength, i.e., ability to promote transcription. Depending upon the host cell system utilized, any one of a number of suitable promoters known in the art is used. For example, for constitutive expression, the CaMV 35S promoter, the rice actin promoter, or the ubiquitin promoter are used. For example, for regulatable expression, the chemically inducible PR-1 promoter from tobacco or *Arabidopsis* is used *(see, e.g.,* U.S. Patent No. 5,689,044).

A preferred category of promoters is that which is wound inducible. Numerous promoters have been described which are expressed at wound sites. Preferred promoters of this kind include those described by Stanford et al. Mol. Gen. Genet. 215: 200-208 (1989), Xu et al. Plant Molec. Biol. 22: 573-588 (1993), Logemann et al. Plant Cell 1: 151-158 (1989), Rohrmeier & Lehle, Plant Molec. Biol. 22: 783-792 (1993), Firek et al. Plant Molec. Biol. 22: 129-142 (1993), and Warner et al. Plant J. 3: 191-201 (1993).

Preferred tissue specific expression patterns include green tissue specific, root specific, stem specific, and flower specific. Promoters suitable for expression in green tissue include many which regulate genes involved in photosynthesis, and many of these have been cloned from both monocotyledons and dicotyledons. A preferred promoter is the maize PEPC promoter from the phosphoenol carboxylase gene (Hudspeth & Grula, Plant Molec. Biol. 12: 579-589 (1989)). A preferred promoter for root specific expression is that described by de Framond (FEBS 290: 103-106 (1991); EP 0 452 269 and a further preferred root-specific promoter is that from the T-1 gene. A preferred stem specific promoter is that described in US patent 5,625,136 and which drives expression of the maize *trpA* gene.

Preferred aspects are transgenic plants expressing nucleotide sequence in a root-specific fashion. Further preferred aspects are transgenic plants expressing the nucleotide sequence in a wound-inducible or pathogen infection-inducible manner.

### 2. Transcriptional Terminators

A variety of transcriptional terminators are available for use in expression cassettes. These are responsible for the termination of transcription beyond the transgene and its correct polyadenylation. Appropriate transcriptional terminators are those that are known to function in plants and include the CaMV 35S terminator, the *tml* terminator, the nopaline synthase terminator and the pea *rbcS* E9 terminator. These are used in both monocotyledonous and dicotyledonous plants.

### 3. Sequences for the Enhancement or Regulation of Expression

Numerous sequences have been found to enhance gene expression from within the transcriptional unit and these sequences can be used in conjunction with the genes to increase their expression in transgenic plants. For example, various intron sequences such as introns of the maize *Adhl* gene have been shown to enhance expression, particularly in monocotyledonous cells. In addition, a number of non-translated leader sequences derived from viruses are also known to enhance expression, and these are particularly effective in dicotyledonous cells.

### 4. Coding Sequence Optimization

The coding sequence of the selected gene may be genetically engineered by altering the coding sequence for optimal expression in the crop species of interest. Methods for modifying coding sequences to achieve optimal expression in a particular crop species are well known (see, *e.g.* Perlak et al., Proc. Natl. Acad. Sci. USA 88: 3324 (1991); and Koziel et al., Bio/technol. 11: 194 (1993)).

In another preferred aspect, a DNA molecule is directly transformed into the plastid genome. Plastid transformation technology is extensively described in U.S. Patent Nos. 5,451,513, 5,545,817, and 5,545,818, in PCT application no. WO 95/16783, and in McBride et al. (1994) Proc. Natl. Acad. Sci. USA 91, 7301-7305. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate homologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab, Z., Hajdukiewicz, P., and Maliga, P. (1990) Proc. Natl. Acad. Sci. USA 87, 8526-8530; Staub, J. M., and Maliga, P. (1992) Plant Cell 4, 39-45). The presence of cloning sites between these markers allowed creation of a plastid targeting vector for introduction of foreign DNA molecules (Staub, J.M., and Maliga, P. (1993) EMBO J. 12, 601-606). Substantial increases in transformation frequency are obtained by replacement of the recessive rRNA or r-protein antibiotic resistance genes with a dominant selectable marker, the bacterial *aadA* gene encoding the spectinomycin-detoxifying enzyme aminoglycoside-3'-adenyltransferase (Svab, Z., and Maliga, P. (1993) Proc. Natl. Acad. Sci. USA 90, 913-917). Previously, this marker had been used successfully for high-frequency transformation of the plastid genome of the green alga *Chlamydomonas reinhardtii* (Goldschmidt-Clermont, M. (1991) Nucl. Acids Res. 19: 4083-4089). Other selectable markers useful for plastid transformation are known in the art .

Plastid expression, in which genes are inserted by homologous recombination into the several thousand copies of the circular plastid genome present in each plant cell. In a preferred aspect, a DNA is inserted into a plastid targeting vector and transformed into the plastid genome of a desired plant host. Plants homoplasmic for plastid genomes containing the DNA molecule are obtained, and are preferentially capable of high expression of the DNA molecule. Preferably, sense and antisense RNA fragments encoded by the DNA molecule are capable of pairing and of forming a double-stranded RNA molecules in plant plastids to to alter the expression of plastid genes. Preferably the sense and antisense fragments do not comprise any mismatch in the complementary region. In another preferred aspect the sense and antisense fragments comprise at least one mismatch in the complementary region. In this case, the DNA sequences in the DNA molecule encoding the RNA fragments are not capable of recombining with each other.

### B. Construction of Plant Transformation Vectors

Numerous transformation vectors available for plant transformation are known to those of ordinary skill in the plant transformation arts, and the genes pertinent to this disclosure can be used in conjunction with any such vectors. The selection of vector depends upon the preferred transformation technique and the target species for transformation. For certain target species, different antibiotic or herbicide selection markers are preferred. Selection markers used routinely in transformation include the *nptII* gene, which confers resistance to kanamycin and related antibiotics (Messing & Vierra. Gene 19: 259-268 (1982); Bevan et al., Nature 304:184-187 (1983)), the *bar* gene, which confers resistance to the herbicide phosphinothricin (White et al., Nucl. Acids Res 18: 1062 (1990), Spencer et al. Theor. Appl. Genet 79: 625-631 (1990)), the *hph* gene, which confers resistance to the antibiotic hygromycin (Blochinger & Diggelmann, Mol Cell Biol 4: 2929-2931), and the *dhfr gene,* which confers resistance to methatrexate (Bourouis et al., EMBO J. 2(7): 1099-1104 (1983)), and the EPSPS gene, which confers resistance to glyphosate (U.S. Patent Nos. 4,940,935 and 5,188,642).

### 1. Vectors Suitable for Agrobacterium Transformation

Many vectors are available for transformation using *Agrobacterium tumefaciens.* These typically carry at least one T-DNA border sequence and include vectors such as pBIN19 (Bevan, Nucl. Acids Res. (1984)) and pXYZ. Typical vectors suitable for *Agrobacterium* transformation include the binary vectors pCIB200 and pCIB2001, as well as the binary vector pCIB10 and hygromycin selection derivatives thereof. (See, for example, U.S. Patent No. 5,639,949).

### 2. Vectors Suitable for non-Agrobacterium Transformation

Transformation without the use of *Agrobacterium tumefaciens* circumvents the requirement for T-DNA sequences in the chosen transformation vector and consequently vectors lacking these sequences are utilized in addition to vectors such as the ones described above which contain T-DNA sequences. Transformation techniques that do not rely on *Agrobacterium* include transformation via particle bombardment, protoplast uptake (*e.g*. PEG and electroporation) and microinjection. The choice of vector depends largely on the preferred selection for the species being transformed. Typical vectors suitable for non-*Agrobacterium* transformation include pCIB3064, pSOG19, and pSOG35. (*See*, for example, U.S. Patent No. 5,639,949).

### C. Transformation Techniques

Once the DNA sequence of interest is cloned into an expression system, it is transformed into a plant cell. Methods for transformation and regeneration of plants are well known in the art. For example, Ti plasmid vectors have been utilized for the delivery of foreign DNA, as well as direct DNA uptake, liposomes, electroporation, micro-injection, and microprojectiles. In addition, bacteria from the genus *Agrobacterium* can be utilized to transform plant cells.

Transformation techniques for dicotyledons are well known in the art and include *Agrobacterium*-based techniques and techniques that do not require *Agrobacterium.* Non-*Agrobacterium* techniques involve the uptake of exogenous genetic material directly by protoplasts or cells. This is accomplished by PEG or electroporation mediated uptake, particle bombardment-mediated delivery, or microinjection. In each case the transformed cells are regenerated to whole plants using standard techniques known in the art. Transformation of most monocotyledon species has now also become routine. Preferred techniques include direct gene transfer into protoplasts using PEG or electroporation techniques, particle bombardment into callus tissue, as well as *Agrobacterium*-mediated transformation.

### EXAMPLES

Standard recombinant DNA and molecular cloning techniques used here are well known in the art and are described by Sambrook, et al., Molecular Cloning, eds., Cold Spring Harbor Laboratory Press, Cold Spring Harbor, NY (1989) and by T.J. Silhavy, M.L. Berman, and L.W. Enquist, Experiments with Gene Fusions, Cold Spring Harbor Laboratory, Cold Spring Harbor, NY (1984) and by Ausubel, F.M. et al., Current Protocols in Molecular Biology, pub. by Greene Publishing Assoc. and Wiley-Interscience (1987).

### Example 1: Regulation of the Expression of a Luciferase Gene (not relating to the invention)

### Construction of a chimeric DNA molecule encoding a luciferase RNA duplex

A 738 bp "sense" oriented fragment of the firefly luciferase gene from plasmid pLuc+ (Promega) is amplified from pPH108 plasmid DNA using oligonucleotide primers ds_Luc1 (5'-CGC GGA TCC TGG AAG ACG CCA AAA ACA-3', SEQ ID NO:1; *BamHI* restriction site underlined) and ds_Luc2 (5'-CGG AAG CTT AGG CTC GCC TAA TCG CAG TAT CCG GAA TG-3', SEQ ID NO:2; *HindIII* restriction site underlined). TurboPfu thermostable DNA polymerise (Stratagene) is used in 50 ul reactions according to the manufacturers protocol with five cycles of 95°C / 1 min, 55°C / 1.5 min, 72°C / 2 min followed by twenty five cycles of 95°C / 1 min, 72°C / 3.5 min. In a similar manner a 737 bp "antisense" oriented fragment of the firefly luciferase gene from plasmid pLuc+ is amplified by PCR from pPH108 plasmid DNA using oligonucleotide primers ds_Luc3 (5'-CGG TCT AGA GGA AGA CGC CAA AAA CAT A-3', SEQ ID NO:3; *XbaI* restriction site underlined) and ds_Luc2 (5'-CGG AAG CTT AGG CTC GCC TAA TCG CAG TAT CCG GAA TG-3', SEQ ID NO: 2; *HindIII* restriction site underlined). The resulting DNA fragments are purified by electrophoresis through a 1 % Tris-acetate gel made from low-melting point agarose (FMC) followed by phenol-chloroform extraction of the excised gel slices containing the PCR products. DNA from the sense product (ds_Luc1/2) is digested with *BamHI* and *Hindlll* and DNA from the antisense product (ds_Luc3/2) is digested with *XbaI* and *HindIII* according to standard methods (restriction enzymes were obtained from New England Biolabs). The resulting sticky-ended DNA fragments are gel purified as described above. A DNA fragment containing the *mas 1'* promoter (Velten et al. (19984) EMBO J. 3: 2723-2730) is obtained by digesting plasmid CSA104 with *EcoRI* and *Hincll* and purifying a 564 bp DNA fragment. This fragment is redigested with *BamHI* and the 484 bp *EcoRI - BamHI* sub-fragment containing the *mas 1'* promoter isolated and gel purified. In order to construct plasmid pPH169, DNA from cloning vector pLitmus29 (New England Biolabs) is digested with *EcoRI* and *XbaI,* and the isolated fragment is ligated in a four-way reaction using T4 DNA ligase (New England Biolabs) to the *mas 1'* promoter *EcoRI - BamHI* fragment and the sense (*BamHI - HindIII*) and antisense (*HindIII - XbaI*) ds_luc luciferase gene fragments. In order to construct binary vector pPH170 for Agrobacterium-mediated plant transformation with the ds_Luc1/2/3 RNA duplex construct, DNA from binary plasmid pSGCHC1 carrying a kanamycin resistance gene for bacterial selection and a hygromycin resistance gene for transgenic plant selection is digested with *EcoRI* and *XbaI.* The resulting 11.6 kb isolated fragment from pSGCHCI is ligated in a four-way reaction using T4 DNA ligase (New England Biolabs) to the *mas 1'* promoter *EcoRI - BamHl* fragment and the sense (*BamHI - HindIII*) and antisense (*HindIII - XbaI*) ds_luc luciferase gene fragments.

### Transformation of Agrobacterium and Vacuum-infiltration of Arabidopsis plants

Plasmids pPH170 is introduced into *Agrbbacterium tumafaciens* GV3101 by electroporation and transformed colonies selected and amplified. Four to five week old plants of *Arabidopsis thaliana* mutant lines expressing luciferase either consitutively (UBQ3 promoter (Norris et al. (1993) PMB 21: 895-906) / UBQ3 + CaMV 35S 5' UTR/luc+; *pPH108*) or inducibly (Arabidopsis PR-1 promoter/luc+; *pPH135, line 6E*) are vacuum infiltrated with Agrobacterium clones carrying the pPH170 binary T-DNA vector. Transformed plants are co-selected on hygromycin and kanamycin and grown under controlled phytotron conditions for determination of luciferase activity. In addition, luciferase activity in the *pPH135*-6E background is assessed 48 hr after induction with BTH (BTH treatment essentially as described in Lawton et al. Plant J. 10: 71-82). Luciferase activity is quantified using a luminescence-based assay in tissue extracts following the addition of luciferin substrate. Luciferase activity is also monitored *in planta* using a CCD-cooled video imaging system (Hamamatsu).

### Example 2: Regulation of the Expression of the Arabidopsis GL1 Gene (not relating to the invention)

The GL1 gene encodes a *myb*-like transcription factor that is required for initiation of normal trichome (leaf hair) formation (Oppenheimer et al. (1991) Cell 67: 483-493). Knock out of GL1 expression early in development results in plants lacking trichomes. The knockout phenotype is easy to identify in young seedlings and is not lethal. Three vectors for constitutive expression and three vectors for Gal4Cl-regulated expression are constructed. The three different vectors to test for each promoter are sense (+) expression, antisense (-) expression, and duplex (+/-) RNA expression of a GL1 gene fragment. The (+) and (-) vectors are controls to compare for their effect on expression of GL1. In each case a 5' fragment from bases #739 to #1781 of the GL1 sequence (GenBank Accession M79448) are used for vector construction.

### Gal4Cl-regulated Expression

The GL1 gene fragments is cloned into the crossing-inducible vector construct pJG304-1 as *NcoI-SacI* fragments. Plasmid pJG304 is derived from pBSSK+. Plasmid pBS SK+ (Stratagene, LaJolla, CA) is linearized with *SacI,* treated with mung bean nuclease to remove the *SacI* site, and re-ligated with T4 ligase to make pJG201. The 10XGAL4 consensus binding site/CaMV 35S minimal promoter/GUS gene/CaMV terminator cassette is removed from pAT71 with *KpnI* and cloned into the *KpnI* site of pJG201 to make pJG304. Plasmid pJG304 is partially digested with restriction endonuclease *Asp718* to isolate a full-length linear fragment. This fragment is ligated with a molar excess of the 22 base oligonucleotide JG-L (5'-GTA CCT CGA G TC TAG ACT CGA G-3', SEQ ID NO: 4). Restriction analysis is used to identify a clone with this linker inserted 5' to the GAL4 DNA binding site, and this plasmid is designated pJG304DXhol.

The *NcoI* and *SacI* sites are added to the ends of (+) and (-) fragments by synthesizing PCR primers with the appropriate restriction sites to the 5' termini. The (+/) GL1 fragment is produced by first producing two fragment : a (+) fragment with the *NcoI* site at the 5' terminus and a *HindIII* site at the 3' terminus and a (-) fragment with a *HindIII* site at the 5' terminus and a *SacI* site at the 3' terminus. The duplex unit is produced by ligation of the resulting fragments at the *EcoRI* site. The expression unit contains the Gal4 DNA binding domain, followed by a minimal TATA sequence, and the GL1 gene fragment oriented either (+), (-) or (+/-).

### Constitutive Expression

The *mas 1'* promoter of mannopine synthase from Agrobacterium(ref), a relatively strong and constitutive in dicot plants is used. As above, the GL (+), (-), and (+/-) fragments are ligated behind the 1' promoter in pBluescript. The three different expression cassettes are ligated into pCIB200 as *EcoRI*/*SaII* fragments (Uknes et al. (1993) Plant Cell 5: 159-169).

### Example 3: Regulation of the Expression of the Cystathionine Beta Lyase Gene (not relating to the invention)

The Cystathionine Beta Lyase (CBL) Gene encodes a step in the methionine biosynthesis pathway. The effect of the regulation of its expression in plants is tested using sense and antisense constructs, and double-stranded RNA constructs.

Antisense construct: binary BASTA vector pJG261 is used containing a fragment from the pJG304DXhol vector with an insertion of part of the CBL gene in an antisense orientation (nucleotides #13-1159, Genbank accession #L40511).

Sense construct: same as antisense construct, except the CBL fragment is in the opposite orientation. This construct contains the ATG start codon and most of the CBL ORF and serves as a control for regulation of the expression of the CBL gene.

Double-stranded RNA construct: A CBL gene fragment (#13-1159) in the sense orientation is inserted into the *SalI* site of vector pJG304-1 downstream of the antisense orientation version of the CBL gene. A linker of about 10 bp is present between the two copies of CBL.

### Example 4: Requirements for Construction of Plant Expression Cassettes

Gene sequences intended for expression in transgenic plants are firstly assembled in expression cassettes behind a suitable promoter and upstream of a suitable transcription terminator. All requirement for constructions of plant expression cassettes apply to the DNA molecules and are carried out using techniques well-known in the art.

### Promoter Selection

The selection of promoter used in expression cassettes determines the spatial and temporal expression pattern of the DNA molecule in the transgenic plant. Selected promoters express DNA molecule in specific cell types (such as leaf epidermal cells, meosphyll cells, root cortex cells) or in specific tissues or organs (roots, leaves or flowers, for example) and this selection reflects the desired location of biosynthesis of a RNA fragment encoded by the DNA molecule. Alternatively, the selected promoter may drive expression of the DNA molecule under a light-induced or other temporally regulated promoter. A further alternative is that the selected promoter be chemically regulated. This provides the possibility of inducing the expression of the DNA molecule only when desired and caused by treatment with a chemical inducer.

### Transcriptional Terminators

A variety of transcriptional terminators are available for use in expression cassettes. These are responsible for the termination of transcription and, preferably, correct polyadenylation. Appropriate transcriptional terminators and those which are known to function in plants and include the CaMV 35S terminator, the *tml* terminator, the nopaline synthase terminator, the pea *rbcS* E9 terminator. These can be used in both monocoylyedons and dicotyledons.

### Sequences for the Enhancement or Regulation of Expression

Numerous sequences have been found to enhance gene expression from within the transcriptional unit and these sequences can be used in conjunction with the DNA molecule to increase its expression in transgenic plants.

Various intron sequences have been shown to enhance expression, particularly in monocotyledonous cells. For example, the introns of the maize *Adh1* gene have been found to significantly enhance the expression of the wild-type gene under its cognate promoter when introduced into maize cells. Intron 1 is found to be particularly effective and enhanced expression in fusion constructs with the chloramphenicol acetyltransferase gene (Callis et al., Genes Develep 1: 1183-1200 (1987)). In the same experimental system, the intron from the maize *bronze 1* gene had a similar effect in enhancing expression (Callis *et al., supra*). Intron sequences have been routinely incorporated into plant transformation vectors, typically within the non-translated leader.

A number of non-translated leader sequences derived from viruses are also known to enhance expression, and these are particularly effective in dicotyledonous cells. Specifically, leader sequences from Tobacco Mosaic Virus (TMV, the "Ω-sequence"), Maize Chlorotic Mottle Virus (MCMV), and Alfalfa Mosaic Virus (AMV) have been shown to be effective in enhancing expression (*e.g*. Gallie et al. Nucl. Acids Res. 15: 8693-8711 (1987); Skuzeski et al. Plant Molec. Biol. 15; 65-79 (1990)).

### Example 5: Examples of Expression Cassette Construction

The present disclosure encompasses the expression of a DNA molecule under the regulation of any promoter which is expressible in plants, regardless of the origin of the promoter. Therefore the DNA molecule is inserted into any of the expression cassette using techniques well-known in the art. These expression cassettes can then be easily transferred to the plant transformation vectors described below Furthermore, the disclosure also encompasses the use of any plant-expressible promoter in conjunction with any further sequences required or selected for the expression of the DNA molecule. Such sequences include, but are not restricted to, transcriptional terminators, extraneous sequences to enhance expression (such as introns [*e.g. Adh* intron 1], viral sequences [*e*. *g*. TMV-Ω]).

### Constitutive Expression: the CaMV 35S Promoter

Construction of the plasmid pCGN1761 is described in the published patent application EP 0 392 225. pCGN1761 contains the "double" 35S promoter and the *tml* transcriptional terminator with a unique *EcoRI* site between the promoter and the terminator and has a pUC-type backbone. A derivative of pCGN1761 is constructed which has a modified polylinker which includes *NotI* and *XhoI* sites in addition to the existing *EcoRI* site. This derivative is designated pCGN1761 ENX. pCGN1761 ENX is useful for the cloning of cDNA sequences or gene sequences (including microbial ORF sequences) within its polylinker for the purposes of their expression under the control of the 35S promoter in transgenic plants. The entire 35S promoter-gene sequence-*tm*/terminator cassette of such a construction can be excised by *Hindlll, Sphl, Sall,* and *XbaI* sites 5' to the promoter and *XbaI, BamHI* and *BglI* sites 3' to the terminator for transfer to transformation vectors. Furthermore, the double 35S promoter fragment can be removed by 5' excision with *HindIII, SphI, SalI, XbaI,* or *PstI,* and 3' excision with any of the polylinker restriction sites (*EcoRI, NotI* or *XhoI*) for replacement with another promoter.

Accordingly, a DNA molecule is inserted into pCGN1761 ENX for constitutive expression under the control of the CaMV 35S promoter.

### Expression under a Chemically Regulatable Promoter

This section describes the replacement of the double 35S promoter in pCGN1761 ENX with any promoter of choice; by way of example the chemically regulated PR-1 a promoter is described. The promoter of choice is preferably excised from its source by restriction enzymes, but can alternatively be PCR-amplified using primers which carry appropriate terminal restriction sites. Should PCR-amplification be undertaken, then the promoter should be resequenced to check for amplification errors after the cloning of the amplified promoter in the target vector. The chemically regulatable tobacco PR-1 a promoter is cleaved from plasmid pCIB1004 (see EP 0 332 104) and transferred to plasmid pCGN1761 ENX. pCIB1004 is cleaved with *NcoI* and the resultant 3' overhang of the linearized fragment is rendered blunt by treatment with T4 DNA polymerase. The fragment is then cleaved with *HindIII* and the resultant PR-1a promoter containing fragment is gel purified and cloned into pCGN1761 ENX from which the double 35S promoter has been removed. This is done by cleavage with *XhoI* and blunting with T4 polymerase, followed by cleavage with *HindIII* and isolation of the larger vector-terminator containing fragment into which the pCIB1004 promoter fragment is cloned. This generates a pCGN1761 ENX derivative with the PR-1 a promoter and the *tmI* terminator and an intervening polylinker with unique *EcoRI* and *NotI* sites.

A DNA molecule of is inserted into this vector, and the fusion product (*i.e*. promoter-gene-terminator) is subsequently transferred to any selected transformation vector, including those described in this application, thus providing for chemically inducible expression of the DNA molecule.

### Constitutive Expression: the Actin Promoter

Several isoforms of actin are known to be expressed in most cell types and consequently the actin promoter is a good choice for a constitutive promoter. In particular, the promoter from the rice *Act1* gene has been cloned and characterized (McElroy et al. Plant Cell 2: 163-171 (1990)). A 1.3 kb fragment of the promoter is found to contain all the regulatory elements required for expression in rice protoplasts. Furthermore, numerous expression vectors based on the *Act1* promoter have been constructed specifically for use in monocotyledons (McElroy et al. Mol. Gen. Genet. 231: 150-160 (1991)). These incorporate the *Act1*-intron 1, *Adh1* 5' flanking sequence and *Adh1*-intron 1 (from the maize alcohol dehydrogenase gene) and sequence from the CaMV 35S promoter. Vectors showing highest expression are fusions of 35S and the *Act1* intron or the *Act1* 5' flanking sequence and the *Act1* intron. The promoter expression cassettes described by McElroy et al. (Mol. Gen. Genet. 231: 150-160 (1991)) is easily modified for the expression of a DNA molecule and are particularly suitable for use in monocotyledonous hosts. For example, promoter containing fragments are removed from the McElroy constructions and used to replace the double 35S promoter in pCGN1761 ENX, which is then available for the insertion or specific gene sequences. The fusion genes thus constructed are transferred to appropriate transformation vectors. In a separate report the rice *Act1* promoter with its first intron has also been found to direct high expression in cultured barley cells (Chibbar et al. Plant Cell Rep. 12: 506-509 (1993)).

A DNA molecule is inserted downstream of such promoter, and the fusion products (*i.e*. promoter-gene-terminator) are subsequently transferred to any selected transformation vector, including those described in this application.

### Constitutive Expression: the Ubiquitin Promoter

Ubiquitin is another gene product known to accumulate in many cell types and its promoter has been cloned from several species for use in transgenic plants (*e.g*. sunflower - Binet et al. Plant Science 79: 87-94 (1991), maize - Christensen et a/. Plant Molec. Biol. 12: 619-632 (1989)). The maize ubiquitin promoter has been developed in transgenic monocot systems and its sequence and vectors constructed for monocot transformation are disclosed in the patent publication EP 0 342 926. Further, Taylor et al. (Plant Cell Rep. 12: 491-495 (1993)) describe a vector (pAHC25) which comprises the maize ubiquitin promoter and first intron and its high activity in cell suspensions of numerous monocotyledons when introduced via microprojectile bombardment. The ubiquitin promoter is clearly suitable for the expression of a DNA molecule in transgenic plants, especially monocotyledons. Suitable vectors are derivatives of pAHC25 or any of the transformation vectors described in this application, modified by the introduction of the appropriate ubiquitin promoter and/or intron sequences.

A DNA molecule is therefore inserted into any of these vector, and the fusion products (*i.e*. promoter-gene-terminator) are used for transformation of plants, resulting in constitutive expression of the DNA molecule.

### Root Specific Expression

A preferred pattern of expression for a DNA molecule is root expression. Expression of the nucleotide sequence only in root tissue has the advantage of altering the expression of a target gene only in roots, without a concomitant alteration of its expression in leaf and flower tissue and seeds. A suitable root promoter is that described by de Framond (FEBS 290: 103-106 (1991)) and also in the published patent application EP 0 452 269. This promoter is transferred to a suitable vector such as pCGN1761ENX and the DNA molecule is inserted into such vector. The entire promoter-gene-terminator cassette is subsequently transferred to a transformation vector of interest.

### Wound Inducible Promoters

Numerous such promoters have been described (*e.g.* Xu et al. Plant Molec. Biol. 22: 573-588 (1993), Logemann et al. Plant Cell 1: 151-158 (1989), Rohrmeier & Lehle, Plant Molec. Biol. 22: 783-792 (1993), Firek et al. Plant Molec. Biol. 22: 129-142 (1993), Warner et al. Plant J. 3: 191-201 (1993)) and all are suitable for use with the instant disclosure Logemann *et al.* (*supra*) describe the 5' upstream sequences of the dicotyledonous potato *wun1* gene. Xu *et al.* (*supra*) show that a wound inducible promoter from the dicotyledon potato (*pin2*) is active in the monocotyledon rice. Further, Rohrmeier & Lehle (*supra*) describe the cloning of the maize *Wip1 cDNA* which is wound induced and which can be used to isolated the cognate promoter using standard techniques. Similarly, Firek *et al.* (*supra*) and Warner *et al.* (*supra*) have described a wound induced gene from the monocotyledon *Asparagus officinalis* which is expressed at local wound and pathogen invasion sites. Using cloning techniques well known in the art, these promoters can be transferred to suitable vectors, fused to a DNA molecule and used to express these genes at the sites of insect pest infection.

### Pith Preferred Expression

Patent application WO 93/07278 describes the isolation of the maize *trpA* gene which is preferentially expressed in pith cells. Using standard molecular biological techniques, this promoter or parts thereof, can be transferred to a vector such as pCGN1761 where it can replace the 35S promoter and be used to drive the expression of a DNA molecule in a pith-preferred manner. In fact, fragments containing the pith-preferred promoter or parts thereof are transferred to any vector and modified for utility in transgenic plants. Pith preferred expression of the DNA molecule is achieved by inserting the DNA molecule in such vector.

### Pollen-Specific Expression

Patent Application WO 93/07278 further describes the isolation of the maize calcium-dependent protein kinase (CDPK) gene which is expressed in pollen cells. The gene sequence and promoter extend up to 1400 bp from the start of transcription. Using standard molecular biological techniques, this promoter or parts thereof, is transferred to a vector such as pCGN1761 where it replaces the 35S promoter and is used to drive the expression of a DNA moleucle in a pollen-specific manner. In fact fragments containing the pollen-specific promoter or parts thereof can be transferred to any vector and modified for utility in transgenic plants.

### Leaf-Specific Expression

A maize gene encoding phosphoenol carboxylase (PEPC) has been described by Hudspeth & Grula (Plant Molec Biol 12: 579-589 (1989)). Using standard molecular biological techniques the promoter for this gene is used to drive the expression of a DNA molecule in a leaf-specific manner in transgenic plants.

### Example 6: Construction of Plant Transformation Vectors

Numerous transformation vectors are available for plant transformation, and a DNA molecule is inserted into any of the expression cassettes described above, such that they are capable of expressing the DNA molecule in desirable cells, under appropriate conditions. A nucleotide sequence-containing expression cassette is then incorporated into any appropriate transformation vector described below.

The selection of vector for use will depend upon the preferred transformation technique and the target species for transformation. For certain target species, different antibiotic or herbicide selection markers may be preferred. Selection markers used routinely in transformation include the *nptII* gene which confers resistance to kanamycin and related antibiotics (Messing & Vierra, Gene 19: 259-268 (1982); Bevan et al., Nature 304:184-187 (1983)), the *bar* gene which confers resistance to the herbicide phosphinothricin (White et al., Nucl Acids Res 18: 1062 (1990), Spencer et al. Theor Appl Genet 79: 625-631(1990)), the *hph* gene which confers resistance to the antibiotic hygromycin (Blochinger & Diggelmann, Mol Cell Biol 4: 2929-2931), and the *dhfr* gene, which confers resistance to methatrexate (Bourouis et al., EMBO J. 2(7): 1099-1104 (1983)).

### Vectors Suitable for Agrobacterium Transformation

Many vectors are available for transformation using *Agrobacterium tumefaciens.* These typically carry at least one T-DNA border sequence and include vectors such as pBIN19 (Bevan, Nucl. Acids Res. (1984)) and pVictor HINK (SEQ ID NO: 5). Below the construction of two typical vectors is described.

### Construction of pCIB200 and pCIB2001

The binary vectors pCIB200 and pCIB2001 are used for the construction of recombinant vectors for use with *Agrobacterium* and is constructed in the following manner. pTJS75kan is created by *NarI* digestion of pTJS75 (Schmidhauser & Helinski, J Bacteriol. 164: 446-455 (1985)) allowing excision of the tetracycline-resistance gene, followed by insertion of an *AccI* fragment from pUC4K carrying an NPTII (Messing & Vierra, Gene 19: 259-268 (1982); Bevan et al., Nature 304: 184-187 (1983); McBride et al., Plant Molecular Biology 14: 266-276 (1990)). *XhoI* linkers are ligated to the *EcoRV fragment* of pCIB7 which contains the left and right T-DNA borders, a plant selectable *nos*/*nptII chimeric* gene and the pUC polylinker (Rothstein et al., Gene 53: 153-161 (1987)), and the *XhoI*-digested fragment is cloned into *SalI*-digested pTJS75kan to create pCIB200 (see also EP 0 332 104). pCIB200 contains the following unique polylinker restriction sites: *EcoRI, SstI, KpnI, BgIIIl, XbaI,* and *SalI. pCIB2001* is a derivative of pCIB200 which created by the insertion into the polylinker of additional restriction sites. Unique restriction sites in the polylinker of pCIB2001 are *EcoRI, Sstl, KpnI, BgIII, Xbal, SaII, MluI, BcII, AvrII, Apal, Hpal,* and *StuI.* pCIB2001, in addition to containing these unique restriction sites also has plant and bacterial kanamycin selection, left and right T-DNA borders for *Agrobacterium*-mediated transformation, the RK2-derived *trfA* function for mobilization between *E. coli* and other hosts, and the *OriT* and *OriV* functions also from RK2. The pCIB2001 polylinker is suitable for the cloning of plant expression cassettes containing their own regulatory signals.
Any one of the plant expression cassettes described above and comprising a DNA molecule are inserted into pCIB2001, preferably using the polylinker.

### Construction of pCIB10 and Hygromycin Selection Derivatives thereof

The binary vector pCIB10 contains a gene encoding kanamycin resistance for selection in plants, T-DNA right and left border sequences and incorporates sequences from the wide host-range plasmid pRK252 allowing it to replicate in both *E. coli* and *Agrobacterium.* Its construction is described by Rothstein et al. (Gene 53: 153-161 (1987)). Various derivatives of pCIB10 have been constructed which incorporate the gene for hygromycin B phosphotransferase described by Gritz et al. (Gene 25: 179-188 (1983)). These derivatives enable selection of transgenic plant cells on hygromycin only (pCIB743), or hygromycin and kanamycin (pCIB715, pCIB717). This vectors is used transform an expression cassette comprising a DNA molecule.

### Vectors Suitable for non-Agrobacterium Transformation

Transformation without the use of *Agrobacterium tumefaciens* circumvents the requirement for T-DNA sequences in the chosen transformation vector and consequently vectors lacking these sequences can be utilized in addition to vectors such as the ones described above which contain T-DNA sequences. Transformation techniques which do not rely on *Agrobacterium* include transformation via particle bombardment, protoplast uptake (*e.g.* PEG and electroporation), microinjection or pollen transformation (US Patent 5,629,183). The choice of vector depends largely on the preferred selection for the species being transformed. Below, the construction of some typical vectors is described.

### Construction of pCIB3064

pCIB3064 is a pUC-derived vector suitable for direct gene transfer techniques in combination with selection by the herbicide basta (or phosphinothricin). The plasmid pCIB246 comprises the CaMV 35S promoter in operational fusion to the *E. coli* GUS gene and the CaMV 35S transcriptional terminator and is described in the PCT published application WO 93/07278. The 35S promoter of this vector contains two ATG sequences 5' of the start site. These sites are mutated using standard PCR techniques in such a way as to remove the ATGs and generate the restriction sites *SspI* and *PvuII.* The new restriction sites are 96 and 37 bp away from the unique *SalI* site and 101 and 42 bp away from the actual start site. The resultant derivative of pCIB246 is designated pCIB3025. The GUS gene is then excised from pCIB3025 by digestion with *SalI* and *SacI,* the termini rendered blunt and religated to generate plasmid pCIB3060. The plasmid pJIT82 is obtained from the John Innes Centre, Norwich and the a 400 bp *SmaI* fragment containing the *bargene* from *Streptomyces viridochromogenes* is excised and inserted into the *HpaI* site of pCIB3060 (Thompson et al. EMBO J 6: 2519-2523 (1987)). This generated pCIB3064 which comprises the bar gene under the control of the CaMV 35S promoter and terminator for herbicide selection, a gene fro ampicillin resistance (for selection in *E. coli*) and a polylinker with the unique sites *Sphl, PstI, HindIII,* and *BamHI.* This vector is suitable for the cloning of plant expression cassettes containing their own regulatory signals to direct expression of a DNA molecule.

### Construction of pSOG19 and pSOG35

pSOG35 is a transformation vector which utilizes the E. *coli* gene dihydrofolate reductase (DHFR) as a selectable marker conferring resistance to methotrexate. PCR is used to amplify the 35S promoter (-800 bp), intron 6 from the maize Adh1 gene (-550 bp) and 18 bp of the GUS untranslated leader sequence from pSOG10. A 250 bp fragment encoding the *E. coli* dihydrofolate reductase type II gene is also amplified by PCR and these two PCR fragments are assembled with a *Sacl-Pstl fragment* from pBI221 (Clontech) which comprised the pUC19 vector backbone and the nopaline synthase terminator. Assembly of these fragments generated pSOG19 which contains the 35S promoter in fusion with the intron 6 sequence, the GUS leader, the DHFR gene and the nopaline synthase terminator. Replacement of the GUS leader in pSOG19 with the leader sequence from Maize Chlorotic Mottle Virus (MCMV) generated the vector pSOG35. pSOG19 and pSOG35 carry the pUC gene for ampicillin resistance and have *Hindlll, Sphl, PstI* and *EcoRI* sites available for the cloning of foreign sequences, in particular a DNA molecule.

### Example 7: Chloroplast Transformation

### Transformation vectors

For expression of a DNA molecule in plant plastids, plastid transformation vector pPH143 (WO 97/32011, example 36) is used. The DNA molecule is inserted into pPH143 thereby replacing the PROTOX coding sequence. This vector is then used for plastid transformation and selection of transformants for spectinomycin resistance. Alternatively, the DNA molecule is inserted in pPH143 so that it replaces the aadH gene. In this case, transformants are selected for resistance to PROTOX inhibitors.

### Chloroplast Transformation

Seeds of *Nicotiana tabacum* c.v. 'Xanthi nc' were germinated seven per plate in a 1" circular array on T agar medium and bombarded 12-14 days after sowing with 1 µm tungsten particles (M10, Biorad, Hercules, CA) coated with DNA from plasmids pPH143 and pPH145 essentially as described (Svab, Z. and Maliga, P. (1993) PNAS 90, 913-917). Bombarded seedlings were incubated on T medium for two days after which leaves were excised and placed abaxial side up in bright light (350-500 µmol photons/m²/s) on plates of RMOP medium (Svab, Z., Hajdukiewicz, P. and Maliga, P. (1990) PNAS 87, 8526-8530) containing 500 µg/ml spectinomycin dihydrochloride (Sigma, St. Louis, MO). Resistant shoots appearing underneath the bleached leaves three to eight weeks after bombardment were subcloned onto the same selective medium, allowed to form callus, and secondary shoots isolated and subcloned. Complete segregation of transformed plastid genome copies (homoplasmicity) in independent subclones was assessed by standard techniques of Southern blotting (Sambrook et al., (1989) Molecular Cloning: A Laboratory Manual, Cold Spring Harbor Laboratory, Cold Spring Harbor). BamHI/EcoRI-digested total cellular DNA (Mettler, I. J. (1987) Plant Mol Biol Reporter 5, 346-349) was separated on 1 % Tris-borate (TBE) agarose gels, transferred to nylon membranes (Amersham) and probed with ³²P-labeled random primed DNA sequences corresponding to a 0.7 kb BamHI/HindIII DNA fragment from pC8 containing a portion of the *rps7*/*12* plastid targeting sequence. Homoplasmic shoots are rooted aseptically on spectinomycin-containing MS/IBA medium (McBride, K. E. et al. (1994) PNAS 91, 7301-7305) and transferred to the greenhouse.

### Example 8: Construction of a chimeric gene cassette encoding a sense and antisense (duplex) RNA fragment for the coat protein gene from BWYV, driven by the RolC promoter (not relating to the invention)

An 0.6 Kb 'sense' oriented fragment of the beet western yellows virus (BWYV), a so-called virus yellow, coat protein (CP) gene is amplified from plasmid pZU046 using primers HINK025btS- (5'-CAA TTA CCA TGG ACA CGG TCG TGG-3'; SEQ ID NO: 6; *NcoI* restriction site underlined), and HiNK226 (5' -GCC AAA TGT TTG AAC GCT GCA GCC TAT TCG-3', SEQ ID NO: 7; *PstI* restriction site underlined). Alternatively the fragment is amplified from plasmid pBW17 (Veidt et al, Nucleic Acids Research 16: 9917-9932, 1988; accession number X13063) using primers HiNK025bis2 (5'-AAT CGT CCA TGG ATA CGG TCG TGG-3', SEQ ID NO: 8; nucleotides 3475 to 3498, *NcoI* restriction site underlined), and HiNK226bis (5'-CTC GGG CCG GGT TCC TCT GCA GCC TAT TTG-3', SEQ ID NO: 9; nucleotides 4114 to 4085, *PstI* restriction site underlined). *Taq* DNA Polymerase (Life Technologies) is used in 25µl reactions according to the manufacturer's prescription applying 30 cycles of 94°C/30 sec, 55°C /30 sec, 72°C/90 sec (+2 sec/cycle). The resulting PCR fragment is purified by electrophoresis through a 1% Tris-acetate gel made from Seakem GTG agarose (FMC), followed by an extraction of the gel slices containing the PCR product with QIAquick Gel Extraction Kit (QIAGEN). The PCR product is subsequently digested with *NcoI* and *PstI* (all restriction enzymes were supplied by Life Technologies) according to standard methods and again gel purified. The purified fragment is ligated between the *RoIC* promoter and the *Nos* terminator using T4 DNA ligase (Life Technologies). The resulting clone is named pHiNK138.

In a similar manner as described above a 1.4 Kb 'antisense' oriented BWYV CP fragment is amplified from plasmid pZU174A using primers HiNK251 (5'-CTC CCA GGT TGA GAC TGC CCT GCA GTG CCC A-3', SEO ID NO: 10; *PstI* restriction site underlined) and HiNK228 (5'-TTA CCA TGC ATA CGG TCG TGG GTA GG-3', SEQ ID NO: 11; *NsiI* restriction site underlined) or from plasmid pBW17 using primers HiNK251 (nucleotides 4844 to 4814) and HiNK228bis (5'-CGT TAA TGC ATA CGG TCG TGG GTA GG-3', SEQ ID NO: 12; nucleotides 3478 to 3503, *NsiI* restriction site underlined). Upon gel purification the PCR product is digested with *NsiI* and *PstI.* The 4.9 Kb plasmid pHiNK138 is linearised with *PstI* and dephosphorylated using the Thermosensitive Alkaline Phosphatase (Life Technologies). Both the vector and the PCR fragment are gel purified, followed by the bidirectional ligation of the PCR fragment into pHiNK138. The orientation generating the duplex RNA for the CP gene (SEQ ID NO: 13) is identified by restriction site analysis, yielding plasmid pHiNK152 in which the inverted repeat consists of the 0.6 Kb CP gene separated by the 0.7 Kb spacer sequence derived from the BWYV genome downstream of the CP gene, referred to as ORF6. The spacer sequence is in the antisense orientation. Finally, the said gene cassette is transferred to the proprietary binary vector pVictorHiNK carrying phosphomannose isomerase as selectable marker gene (WO 94/20627), yielding pHiNK179.

### Example 9.1: Construction of a chimeric gene cassette encoding a sense and antisense (duplex) RNA fragment for the replicase gene from BNYVV, driven by the Arabidopsis Ubi3int promoter

Total RNA is extracted from sugar beet root infected by the beet necrotic yellow vein virus (BNYVV), a furovirus, using the RNAeasy Plant mini kit from QIAGEN. In order to amplify the 3' end of the BNYVV replicase gene (RNA1) the RNA is reverse transcribed to produce a cDNA using the Superscript^{™}II RNAse H-Reverse Transcriptase (RT) (Life Technologies) and the reverse primer HiNK285 (5'-TCG TAG AAG AG A ATT CAC CCA AAC TAT CC-3', SEQ ID NO: 14). Primer HiNK285 is located between nucleotides 6378 and 6405 of the BNYVV RNA1 sequence (accession number D00115) and designed to introduce an *EcoRI* site. The RT reaction is subsequently used as template for two PCR reactions :
- Reaction A using primer HiNK283 (5' -AAG AAT TGC AGG A_{T}C CAC AGG CTC GGT AC-3', SEQ ID NO: 15) located between nucleotides 5168 bp and 5178 bp of BNYVV RNA1 designed to introduce a *BamHI* site, and primer HiNK284 (5' -TTC CAA CGA ATT CGG TCT CAG AC A-3', SEQ ID NO: 16) located between nucleotides 5597 and 5620 of BNYVV RNA1 designed to introduce an *EcoRI* site.
- Reaction B using primer HiNK283 in combination with primer HiNK285, both described above.

The thus obtained RT-PCR products share the BNYVV RNA1 sequence between nucleotides 5168-5620 that constitutes the future RNA duplex. The future spacer sequence corresponds to nucleotides 5621-6405 bp of BNYVV RNA1 present at the RT-PCR product obtained with primers HiNK283 and HiNK285.

*Taq* DNA Polymerase (Life Technologies) is used in 25µl reactions according to the suppliers's prescription applying 30 cycles of 94°C/30 sec, 55°C/30 sec, 72°C/90 sec(+2 sec/cycle). The resulting RT-PCR products are purified by electrophoresis through a 1% Tris-acetate gel made from Seakem GTG agarose (FMC), followed by an extraction of the gel slices containing the amplification products with the QIAquick Gel Extraction Kit (QIAGEN). After gel purification, the RT-PCR products are digested with restriction enzymes *BamHI* and *EcoRI* (Life Technologies) according to standard methods and purified as described above.

Finally the RT-PCR products are cloned between the *Arabidopsis* Ubiquitin3 *(Ubi3int)* promoter and the *Nos* terminator by means of a a three-way ligation reaction using T4 DNA ligase (Life Technologies). The two resulting clones are named pHiNK181 (spacer in antisense orientation, see SEQ ID NO: 17) and pHiNK184 (spacer in sense orientation, see SEQ ID NO: 18).

In order to construct the binary vectors for the *Agrobacterium*-mediated transformation of sugar beet, DNA from binary vector pVictorHiNK carrying the phophomannose isomerase gene as selectable marker (WO 94/20627), as well as plasmids pHiNK181 and pHiNK184, are digested with with *AscI* and *PacI* (New England Biolabs) and the vector and insert fragments purified by electrophoresis as described above. The resulting 7.7 kb pVictorHiNK vector fragment is ligated using T4 DNA ligase (Life Technologies) to the gene cassettes encoding the duplex RNA for the BNYVV replicase gene yielding pHiNK187 (antisense spacer) and pHiNK188 (sense spacer) respectively.

### Example 9.2: Agrobacterium-mediated transformation of sugar beet

Methods for the *Agrobacterium*-mediated transformation of plants species are well established and known to a person skilled in the art, and may vary for the explant type, the *Agrobacterium* strain, or the selectable marker and regeneration system used. The protocol here below describes the Agrobacterium-mediated transformation of sugar beet using cotyledons as source for explants and mannose-6-phosphate isomerase as selectable marker gene (Joersbo et al, Molecular Breeding 4: 111-117, 1998). Upon surface sterilization sugar beet seeds are germinated on water agar and under dim light at a temperature of approximately 12°C. Fully developed cotyledons are removed from the seedling by a transversal cut just below the nodal region and both cotelydons torn apart by gentle pulling and cutting. Cotyledon explants are inoculated by dipping them into a suspension of *Agrobacterium* strain EHA101 carrying the appropriate transformation vector diluted in MS medium pH 5.2, supplemented with 20 g/l sucrose, 0.25 mg/l BA, 0.05 mg/l NAA, 500 µM acetosyringone diluted to an optical density (OD600) from 0.1 to 0.3. After five minutes of incubation explants are removed from the *Agrobacterium* suspension, dipped on sterile filter paper to remove access of the inoculation suspension, and transferred to co-cultivation plates. The co-cultivation plates consist of petri dishes containing 1/10 MS medium pH 5.7, 30 g/l sucrose, 200 µM acetosyringone, solidified with 4.7 g/l agarose and covered with a filter paper moistened with 1.5 ml TXD medium (MS salts supplemented with PGO vitamins, 0.005 mg/l kinetin, 4 mg/l CPA (p-chlorophenoxy acetic acid), 30 g/l sucrose, pH 5.7) on the top of the solidified medium. Explants are co-cultivated during 4 days at 21°C and under dim light, and subsequently transferred to selective regeneration medium consisting of MS medium pH 5.9 supplemented with 20 g/l sucrose, 1.25 g/l mannose, 0.25 mg/l BA, 0.05 mg/l NAA, 500 mg/l carbenicillin, and solidified with 9 g/l agar. Every third week the explants are subcultured to fresh media containing gradually increasing concentrations of mannose to a maximum of 15 g/l. After 12 weeks of selection and regeneration at a temperature of 21 °C, regenerated shoots are harvested, rooted and analyzed for PMI activity essentially according to the coupled enzyme assay described by Feramisco and co-workers (Feramisco et al, Biochem. Biophys. Res. Comm. 55: 636-641, 1973). Positive plants are potted in soil and finally transferred to the greenhouse.

### Example 9.3: Screening for resistance to rhizomania

Upon sowing or potting to soil, seedlings or T0 transformants are first allowed to establish a sufficiently developed root system for a period of approximately four weeks, prior to their transfer to soil infested with *Polymyxa betae* carrying BNYVV. Infested soils are collected from rhizomania infected fields in Germany. During the resistance assay plants are grown in 12 cm pots at a temperature of approximately 21°C and light period of 16 hours. Four weeks after transplantation into the infested soil, plants are pulled from the soil and the bottom half of the root system cleaned from any adhering dirt by rinsing with water. Sap from a random sample of 0.5 g of root tissue is collected by means of a Pollähne press and added to 10 ml of extraction buffer consisting of phosphate buffered saline pH 7.2, supplemented with 2% PVP and 0.2% ovalbumin. The amount of virus present in the samples (clones are obtained by *in vitro* propagation) is determined by means of the triple antibody sandwich (TAS) ELISA for BNYVV commercialized by Adgen Ltd, Scotland, UK, essentially following the supplier's instruction. A standard curve is included on each plate to calculate the virus content of the root samples from the measured absorbance values. Non-transformed susceptible sugar beet plants serve a negative controls, plants carrying the C28 gene for natural rhizomania resistance as positive controls. Table 1 summarizes the results of transformation events obtained with plasmid pHINK188.

**Table 1:**

| **Event** | **Clone number** | **ELISA value** | **Virus content ng/ml** |
|---|---|---|---|
| Event 279-15-A | 1 | 3.265 | >900 |
| | 2 | 3.420 | >900 |
| | 3 | 2.987 | >900 |
| Event 284-22-A | 1 | 0.119 | 4 |
| Event 284-22-G | 1 | 0.022 | 0 |
| | 2 | 0.006 | 0 |
| Event 284-22-I | 1 | 0.010 | 0 |
| Event 284-22-M | 1 | 1.400 | 216 |
| | 2 | 1.127 | 138 |
| Event 284-22-Q | 1 | 0.447 | 29 |
| | 2 | 0.049 | 1 |
| Event 284-22-U | 1 | 3.420 | >900 |
| | 2 | 3.582 | >900 |
| Event 284-22-1 F | 1 | 3.945 | >900 |
| | 2 | 0.131 | 4 |
| C28 positive control | 1 | 0.677 | 56 |
| | 2 | 0.679 | 56 |
| | 3 | 0.377 | 22 |
| | 4 | 0.756 | 67 |
| | 5 | 1.122 | 137 |
| | 6 | 0.640 | 51 |
| Negative control A | 1 | 3.186 | >900 |
| | 2 | 3.362 | >900 |
| | 3 | 3.487 | >900 |
| | 4 | 3.311 | >900 |
| | 5 | 3.478 | >900 |
| Negative control B | 1 | 3.678 | >900 |
| | 2 | 3.582 | >900 |
| | 3 | 3.326 | >900 |
| | 4 | 3.502 | >900 |
| | 5 | 3.330 | >900 |
| | 6 | 3.439 | >900 |
| | 7 | 3.439 | >900 |
| | 8 | 3.682 | >900 |

### Example 10: Construction of a plant transformation vector for Zucchini yellow mosaic potyvirus (ZYMV) and Papaya ringspot potyvirus (PRSV) resistance in Melon: (not relating to the invention)

The NOS terminator (Bevan, M., et al, 1983 Nucleic Acids Res. 11 (2), 369-385) is cloned as 260 bp HindIII/PstI fragment into the plasmid pZO1560 digested with HindIII/PstI. PZO1560 is a pUC derived plasmid in which the multiple cloning site has been replaced by a more versatile one. The new construct obtained after insertion of the NOS terminator is named pZU533.

The 1728 bp Ubi3 promoter/intron fragment (Callis, J., et al, (1995 Genetics 139 (2), 921-939) is isolated by digestion with BamHI followed by a T4 DNA polymerase treatment and KpnI digestion. This fragment is cloned into pZU533 digested with Smal. The new construct is named pZU615.

Additional DNA fragments to be inserted into pZU615 are firstly amplified by PCR using Pfx DNA polymerase and the PCR fragments obtained are cloned into pBluescript SK+.

To amplify a 513 bp Actin2 IntronL (leader intron) fragment two primers are designed based on the sequence from An et al, The Plant Journal: 10: 107-121, 1996. The 5' primer (ZUP1563: 5'-GGGCGGATCCGCTAGCCCGCGGCCGCTCTTTCTTTCCAAGG-3', SEQ ID NO: 19) anneals directly upstream of the 5' splice site and adds a BamHI, a Nhel and a SacII restricion site to the 5'-end of the Actin2 IntronL fragment to be amplified. The 3' primer (ZUP1564: 5'-CCCGCCATGGGTCGACGCCATTTTTTATGAGCTGC-3', SEQ ID NO: 20) anneals directly downstream of the 3' splice site and adds a SaII and a Ncol restricion site to the 3'-end of the Actin2 IntronL fragment to be amplified. The PCR fragment amplified with ZUP1563 and 1564 is cloned in the EcoRV site of pBluescript. The new plasmid is named pZU611. To provide plasmid pZU615 with the Act2 IntronL a 499 bp BamH/Ncol Act2 IntronL fragment is isolated from pZU611 and cloned in pZU615 digested with BamHI and Ncol downstream of the Ubi3 promoter/intron and upstream of the NOS terminator. The new construct is designated pZU616. The insertion of this fragment simultaneously provides the cassette with five additional restriction sites for the next three cloning steps.

To amplify a non-translated (nt) 739 bp PRSV CP fragment (Shyi-Dong, Y., et al, (1992). Journal of General Virology 73, 2531-2541) two primers are designed based on the sequence of a French field isolate from Shyi-Dong Yeh. The 5' primer (ZUP1565: 5'-CCCGCCATGGGATCCGATGATTTCTACCGAGAATTAAGGG -3', SEQ ID NO: 21) anneals 285 bp downstream of the start codon of PRSV CP and adds a Ncol and a BamHI restricion site to the 5'-end of the non-translated PRSV CP fragment. The 3' primer (ZUP1566: 5'-GGGCGCTAGCCTAATGCTTATATAGTACC-3', SEQ ID NO: 22) anneals 55 bp downstream of the PRSV CP stop codon and adds a Nhel restricion site to the 3'-end of the non-translated PRSV CP fragment. The amplified PCR fragment is cloned in the EcoRV site of pBluescript and the new plasmid is designated pZU612.

To amplify a 735 bp non-translated ZYMV CP fragment (Gal-On, A., et al, (1990). Gene 87, 273-277) two primers are designed based on the sequence of a French field isolate from Gal-On, A.. The 5' primer (ZUP1567: 5'- GGGCGCTAGCCTTGCTGGAGTATAAGCCGG-3', SEQ ID NO: 23) anneals 253 bp downstream of the start codon of ZYMV CP and includes a Nhel restricion site at the 5'-end of the ZYMV CP non-translated fragment. The 3' primer (ZUP1568: 5'- GGGCGTCGACCGCGGGCTTTAAAGGTGGGAGGCCC -3', SEQ ID NO: 24) anneals 89 bp downstream of the ZYMV CP stop codon and includes SacII and a SalI restricion sites at the 3' end of the non-translated ZYMV CP fragment. The amplified PCR fragment is cloned in the EcoRV site of pBluescript. The new plasmid is named pZU613. A ZYMV CP non-translated fragment is subsequently cloned into pZU616 downstream of the Ubi3 promoter/intron and upstream of the Act2 IntronL. For this purpose a 719 bp NheI/SacII ZYMV CP non-translated fragment is isolated from pZU613 and cloned in the Nhel and SacII sites of pZU616. The construct is designated pZU617. Subsequently a PRSV CP non-translated fragment is cloned into pZU617 downstream of the Ubi3 promoter/intron and upstream of the ZYMV CP non-translated fragment. For this purpose a 720 bp BamHI/NheI PRSV CP non-translated fragment is isolated from pZU612 and cloned in the BamHI and Nhel sites of pZU617. This new construct is named pZU618.

The last fragment to be cloned to complete the gene cassette contains a PRSV CP non-translated fragment and downstream thereof a ZYMV CP non-translated fragment in the same orientation. A corresponding fragment is amplified from pZU618 as template using Pfx PCR and the primers ZUP1565 and ZUP1568. The PCR fragment is cloned into the EcoRV site of pBluescript SK+ and the resulting plasmid is named pZU619.

To create an inverted repeat gene cassette the PRSV CP (nt) / ZYMV CP (nt) fragment is inserted in pZU618 in opposite direction of the PRSV CP (nt) / ZYMV CP (nt) fragments already present in the pZU618 plasmid. For this purpose a 1445 bp SalI/NcoI PRSV CP (nt) / ZYMV CP (nt) fragment is isolated from pZU619 and cloned in pZU618 digested with SalI and NcoI downstream of the Act2 IntronL and upstream of the NOS terminator. The resulting plasmid is designated pZU622. Said cassette is inserted into the binary vector pZU547, a binary vector derived from the plasmid pVictorHink additionally containing a SMAS promoter/ PMI /NOS terminator selection cassette. To this end the 5414 bp AscI/PacI DNA fragment containing the inverted repeat gene cassette is isolated from pZU622 and cloned into the AscI and PacI sites of pZU547 in tandem orientation and upstream of the selection cassette. The final construct is designated pZU623 (SEQ ID NO: 25).

### Example 11: Construction of a plant transformation vector for Potato Virus Y (PVY) resistance in Tomato (not relating to the invention)

Based on the sequence of PVYn (a French field isolate of PVY), published in the thesis 'Engineering resistance against potato virus Y' of R.A.A. van der Vlugt (1993), two primers (ZUP1598: 5'-CATGCCATGGATCCAATGGCCACGAATTAAAGCTATCACGTC-3'- SEQ ID NO: 26, and ZUP1590: 5'-ACGCGTCGACCGCGGATTCAAACGATTATTAATTACGATAAAAG-3'-SEQ ID NO: 27) are used to amplify by standard PCR techniques using Platinum Pfx DNA polymerase from Life Technologies a 804 bp fragment containing the coat protein cistron sequences and 99 nucleotides of the 3'-end non-translated region. The amplified PCR fragment is cloned as a blunt end fragment in the EcoRV site of pBluescript SK+ the plasmid being designated pZUA. The amplified PVY specific insert is excised from as a BamHI/SacII fragment and cloned into the BamHI/SacII sites of pZU616 (see exmaple 10), yielding pZUB. PZUB and pZUA are both digested with NcoI and SalI. The PVY specific fragment from pZUA is purified from agarose gel and ligated into the pZUB digested with NcoI and SalI, yielding pZUC containing the following elements:

The UBI3 promoter with intron followed by the PVY PCR product in sense orientation as a BamHI/SaclI fragment, followed by a SacII/SalI ACT2 intron, followed by the PVY PCR product in antisense orientation as a Sall/Ncol fragment and finally a nos terminator as a NcoI/HindIII fragment. Finally, pZUC is cloned into the binary vector pZU547 containing the SMAS promoter / PMI / NOS terminator selection cassette derived from the pHiNK 085 binary vector. The final construct is designated pZU634 (SEQ ID NO: 28).

### Example 12: Transformation of binary vectors to melon and tomato plant material (not relating to the invention)

Methods to transfer binary vectors to plant material are well established and known to a person skilled in the art. Variations in the procedures are due to, for instance, differences in *Agrobacterium* strains used, different sources of explant material, differences between the regeneration systems, as well as different cultivars of the plant species to be transformed. The binary plant transformation vectors described in examples 10 and 11 above are used in transformation experiments according to the following procedures. The binary vectors are transferred to *Agrobacterium tumefaciens* by electroporation, followed by inoculation and cocultivation of plant explant material with the transformed *Agrobacterium* strain, selective killing of the *Agrobacterium* strain using an appropriate antibiotic, selection of transformed cells by growing on selective media containing mannose, transfer of tissue to shoot inducing media, transfer of selected shoots to root inducing media, and transfer of plantlets to soil. To confirm the presence of the gene cassettes described in examples 10 and 11 above, total DNA from the transgenic plants is characterized using well known Southern Blot Analysis techniques.

### Example 13: Screening transgenic melon plants for ZYMV and PRSV resistance (not relating to the invention)

Transformed plants are grown in the greenhouse under standard quarantine conditions in order to prevent any infections by pathogens. Primary transformants are self pollinated and seeds harvested.

100 plants of the S1 progeny of the primary transformants are analyzed for segregation of the inserted gene and subsequently infected with ZYMV by mechanical inoculation. Tissue from host plants systemically infected with ZYMV is ground in 5 volumes of ice cold inoculation buffer (10mM phosphate buffer) and rubbed in the presence of carborundum powder on the cotelydons and first leave of 1 week old seedlings. Inoculated plants are monitored for symptom development during 3 weeks after inoculation. Plants containing ZYMV sequences show reduced susceptibility to ZYMV infection compared with untransformed control plants which show severe systemic ZYMV symptoms within 7 days after inoculation. ZYMV tolerant plants are self pollinated and seeds harvested. Transgenic ZYMV resistant plants are mechanically inoculated with PRSV according to the procedures described above. Plants already resistant to ZYMV also show reduced susceptibility to PRSV infection compared with untransformed control plants which show severe systemic PRSV symptoms within 7 days after inoculation.

### Example 14: Screening of transgenic tomato plants for resistance against PVY (not relating to the invention)

Transformed plants are grown in the greenhouse under standard quarantine conditions in order to prevent any infections by pathogens. Primary transformants are self pollinated and seeds harvested.

50 plants of the S1 progeny of the primary transformants are analyzed for segregation of the inserted gene and subsequenly infected with PVY by mechanical inoculation. Tissue from host plants systemically infected with PVY is ground in 5 volumes of ice cold inoculation buffer (10mM phosphate buffer) and rubbed in the presence of carborundum powder on the first two fully extended leaves of 5 weeks old seedlings. Inoculated plants are monitored for symptom development during 3 weeks after inoculation. Plants containing PVY sequences show reduced susceptibility to PVY infection compared with untransformed control plants which show severe systemic PVY symptoms within 7 days after inoculation.

## Claims

1. A method of altering the expression of a viral target gene comprising introducing into a cell a first DNA sequence capable of expressing in said cell a sense RNA fragment of a viral target gene and a second DNA sequence capable of expressing in said cell an antisense RNA fragment of said target gene, wherein said sense RNA fragment and said antisense RNA fragment are capable of forming a double-stranded RNA molecule, wherein said cell is a plant cell which is rendered virus resistant or tolerant, said virus is a furovirus and said target gene is a replicase gene or portion thereof.

2. The method of claim 1, wherein said first DNA sequence and said second DNA sequence are stably integrated in the genome of said cell.

3. The method of claim 1 , wherein said first DNA sequence and said second DNA sequence are comprised in two separate DNA molecules.

4. The method of claim 1, wherein at least two pairs of first and second DNA sequences are introduced into a cell.

5. The method of claim 4, wherein each pair of DNA sequences encodes sense and antisense RNA fragments of different virus species or isolates.

6. The method of claim 1, wherein said first DNA sequence and said second DNA sequence are comprised in one DNA molecule.

7. The method of claim 6, wherein said first DNA sequence and said second DNA sequence are comprised in the same DNA strand of said DNA molecule.

8. The method of claim 7, wherein said sense RNA fragment and said antisense RNA fragment are comprised in one RNA molecule.

9. The method of claim 8, wherein said RNA molecule is capable of folding such that said RNA fragments comprised therein form a double-stranded region.

10. The method of claim 1, wherein the the expressed RNA molecule comprises the inverted repeat sequence of SEQ ID NO: 17 or SEQ ID NO: 18.

11. The method of claim 10, wherein expression of said RNA molecule is driven by a heterologous, a tissue-specific, a developmentally regulated, a constitutive, or an inducible promoter.

12. The method of claim 11, wherein expression of said RNA molecule is driven by a ubiquitin promoter such as the Arabidopsis Ubi3 promoter.

13. The method of claim 8, wherein said DNA molecule comprises a linker between the DNA sequences encoding said sense RNA fragment and said antisense RNA fragments.

14. The method of claim 13, wherein said linker comprises an expression cassette of a functional gene such as a selectable marker gene.

15. The method of claim 14, wherein said linker comprises regulatory sequences such as intron processing signals.

16. The method of claim 7, wherein said sense RNA fragment and said antisense RNA fragment are comprised in two separate RNA molecules.

17. The method of claim 16, wherein said first DNA sequence and said second DNA sequence are operably linked to a bi-directional promoter.

18. The method of claim 7, wherein said first DNA sequence and said second DNA sequence are comprised in complementary strands of said DNA molecule.

19. The method of claim 18, wherein said first DNA sequence is the complementary DNA strand of said second DNA sequence in said DNA molecule.

20. A DNA construct altering the expression of a viral genome or a portion thereof comprising a first DNA sequence capable of expressing in a cell a sense RNA fragment of said viral genome and a second DNA sequence capable of expressing in said cell an antisense RNA fragment of said viral genome, wherein said sense RNA fragment and said antisense RNA fragment are capable of forming a double-stranded RNA molecule wherein said cell is a plant cell which is rendered virus resistant or tolerant, said virus is a furovirus and said DNA sequences comprise a replicase gene or portions thereof.

21. The DNA construct of claim 20, wherein said DNA construct comprises a first promoter operably linked to said first DNA sequence and a second promoter operably linked to said second DNA sequence.

22. The DNA construct of claim 20, wherein said DNA construct comprises a bi-directional promoter operably linked to said first DNA sequence and to said second DNA sequence.

23. A cell showing altered expression of a viral target gene comprising a first DNA sequence capable of expressing in said cell a sense RNA fragment of said viral target gene and a second DNA sequence capable of expressing in said cell an antisense RNA fragment of said viral target gene, wherein said sense RNA fragment and said antisense RNA fragment are capable of forming a double-stranded RNA molecule, and wherein said cell is a plant cell which is rendered virus resistant or tolerant, said virus is a furovirus and said target gene is a replicase gene or portion thereof.

24. A plant and the progeny thereof comprising the plant cell of claim 23.

25. The plant of claim 24, wherein said plant is virus resistant or tolerant.

26. Seeds comprising the plant cell of claim 23.

## Patentansprüche

1. Verfahren zur Veränderung der Expression eines viralen Zielgens, bei dem man in eine Zelle eine erste DNA-Sequenz mit der Fähigkeit zur Expression eines Sense-RNA-Fragments eines viralen Zielgens in der Zelle und eine zweite DNA-Sequenz mit der Fähigkeit zur Expression eines Antisense-RNA-Fragments des Zielgens in der Zelle einführt, wobei das Sense-RNA-Fragment und das Antisense-RNA-Fragment ein doppelsträngiges RNA-Molekül bilden können, wobei es sich bei der Zelle um eine Pflanzenzelle, die virusresistent oder -tolerant gemacht wird, bei dem Virus um ein Furovirus und bei dem Zielgen um ein Replikase-Gen oder einen Teil davon handelt.

2. Verfahren nach Anspruch 1, wobei die erste DNA-Sequenz und die zweite DNA-Sequenz in dem Genom der Zelle stabil integriert sind.

3. Verfahren nach Anspruch 1, wobei die erste DNA-Sequenz und die zweite DNA-Sequenz in zwei getrennten DNA-Molekülen enthalten sind.

4. Verfahren nach Anspruch 1, wobei wenigstens zwei Paare erster und zweiter DNA-Sequenzen in eine Zelle eingeführt werden.

5. Verfahren nach Anspruch 4, wobei die Paare von DNA-Sequenzen jeweils für Sense- und Antisense-RNA-Fragmente unterschiedlicher Virusspezies oder -isolate codieren.

6. Verfahren nach Anspruch 1, wobei die erste DNA-Sequenz und die zweite DNA-Sequenz in einem DNA-Molekül enthalten sind.

7. Verfahren nach Anspruch 6, wobei die erste DNA-Sequenz und die zweite DNA-Sequenz im gleichen DNA-Strang des DNA-Moleküls enthalten sind.

8. Verfahren nach Anspruch 7, wobei das Sense-RNA-Fragment und das Antisense-RNA-Fragment in einem RNA-Molekül enthalten sind.

9. Verfahren nach Anspruch 8, wobei sich das RNA-Molekül so falten kann, dass die darin enthaltenen RNA-Fragmente einen doppelsträngigen Bereich bilden.

10. Verfahren nach Anspruch 1, wobei das exprimierte RNA-Molekül die Inverted-Repeat-Sequenz der SEQ ID NO: 17 oder SEQ ID NO: 18 umfasst.

11. Verfahren nach Anspruch 10, wobei die Expression des RNA-Moleküls durch einen heterologen, einen gewebespezifischen, einen entwicklungsregulierten, einen konstitutiven oder einen induzierbaren Promotor angetrieben wird.

12. Verfahren nach Anspruch 11, wobei die Expression des RNA-Moleküls durch einen Ubiquitin-Promotor, wie etwa den Arabidopsis-Ubi3-Promotor, angetrieben wird.

13. Verfahren nach Anspruch 8, wobei das DNA-Molekül einen Linker zwischen den für das Sense-RNA-Fragment und das Antisense-RNA-Fragment codierenden DNA-Sequenzen umfasst.

14. Verfahren nach Anspruch 13, wobei der Linker eine Expressionskassette eines funktionellen Gens, wie etwa eines selektionierbaren Marker-Gens, umfasst.

15. Verfahren nach Anspruch 14, wobei der Linker regulatorische Sequenzen, wie etwa Intronprozessierungssignale, umfasst.

16. Verfahren nach Anspruch 7, wobei das Sense-RNA-Fragment und das Antisense-RNA-Fragment in zwei getrennten RNA-Molekülen enthalten sind.

17. Verfahren nach Anspruch 16, wobei die erste DNA-Sequenz und die zweite DNA-Sequenz in operativer Verknüpfung mit einem bidirektionalen Promotor stehen.

18. Verfahren nach Anspruch 7, wobei die erste DNA-Sequenz und die zweite DNA-Sequenz in komplementären Strängen des DNA-Moleküls enthalten sind.

19. Verfahren nach Anspruch 18, wobei es sich bei der ersten DNA-Sequenz um den komplementären DNA-Strang der zweiten DNA-Sequenz in dem DNA-Molekül handelt.

20. DNA-Konstrukt, das die Expression eines Virusgenoms oder eines Teils davon verändert, umfassend eine erste DNA-Sequenz mit der Fähigkeit zur Expression eines Sense-RNA-Fragments des Virusgenoms in einer Zelle und eine zweite DNA-Sequenz mit der Fähigkeit zur Expression eines Antisense-RNA-Fragments des Virusgenoms in der Zelle, wobei das Sense-RNA-Fragment und das Antisense-RNA-Fragment ein doppelsträngiges RNA-Molekül bilden können, wobei es sich bei der Zelle um eine Pflanzenzelle, die virusresistent oder -tolerant gemacht wird, und bei dem Virus um ein Furovirus handelt und die DNA-Sequenzen ein Replikase-Gen oder Teile davon umfassen.

21. DNA-Konstrukt nach Anspruch 20, wobei das DNA-Konstrukt einen mit der ersten DNA-Sequenz in operativer Verknüpfung stehenden ersten Promotor und einen mit der zweiten DNA-Sequenz in operativer Verknüpfung stehenden zweiten Promotor umfasst.

22. DNA-Konstrukt nach Anspruch 20, wobei das DNA-Konstrukt einen mit der ersten DNA-Sequenz und mit der zweiten DNA-Sequenz in operativer Verknüpfung stehenden bidirektionalen Promotor umfasst.

23. Zelle, die eine veränderte Expression eines viralen Zielgens zeigt, das eine erste DNA-Sequenz mit der Fähigkeit zur Expression eines Sense-RNA-Fragments des viralen Zielgens in der Zelle und eine zweite DNA-Sequenz mit der Fähigkeit zur Expression eines Antisense-RNA-Fragments des viralen Zielgens in der Zelle umfasst, wobei das Sense-RNA-Fragment und das Antisense-RNA-Fragment ein doppelsträngiges RNA-Molekül bilden können und wobei es sich bei der Zelle um eine Pflanzenzelle, die virusresistent oder -tolerant gemacht wird, bei dem Virus um ein Furovirus und bei dem Zielgen um ein Replikase-Gen oder einen Teil davon handelt.

24. Pflanze und Nachkommenschaft davon, umfassend die Pflanzenzelle nach Anspruch 23.

25. Pflanze nach Anspruch 24, wobei die Pflanze virusresistent oder -tolerant ist.

26. Saatgut, umfassend die Pflanzenzelle nach Anspruch 23.

## Revendications

1. Procédé pour altérer l'expression d'un gène cible viral, comprenant l'introduction, dans une cellule, d'une première séquence d'ADN capable d'exprimer dans ladite cellule un fragment d'ARN sens d'un gène cible viral et d'une deuxième séquence d'ADN capable d'exprimer dans ladite cellule un fragment d'ARN antisens dudit gène cible, dans lequel ledit fragment d'ARN sens et ledit fragment d'ARN antisens sont capables de former une molécule d'ARN double brin, ladite cellule étant une cellule végétale qui est rendue résistante ou tolérante à un virus, ledit virus étant un furovirus et ledit gène cible étant un gène de la réplicase ou une portion de ce dernier.

2. Procédé de la revendication 1, dans lequel ladite première séquence d'ADN et ladite deuxième séquence d'ADN sont intégrées de manière stable dans le génome de ladite cellule.

3. Procédé de la revendication 1, dans lequel ladite première séquence d'ADN et ladite deuxième séquence d'ADN sont présentes dans deux molécules d'ADN distinctes.

4. Procédé de la revendication 1, dans lequel au moins deux paires de la première et de la deuxième séquences d'ADN sont introduites dans une cellule.

5. Procédé de la revendication 4, dans lequel chaque paire de séquences d'ADN code pour des fragments d'ARN sens et antisens de différentes espèces virales ou de différents isolats viraux.

6. Procédé de la revendication 1, dans lequel ladite première séquence d'ADN et ladite deuxième séquence d'ADN sont présentes dans une molécule d'ADN.

7. Procédé de la revendication 6, dans lequel ladite première séquence d'ADN et ladite deuxième séquence d'ADN sont présentes dans le même brin d'ADN de ladite molécule d'ADN.

8. Procédé de la revendication 7, dans lequel ledit fragment d'ARN sens et ledit fragment d'ARN antisens sont présents dans une molécule d'ARN.

9. Procédé de la revendication 8, dans lequel ladite molécule d'ARN est capable de se replier de telle sorte que lesdits fragments d'ARN qui y sont présents forment une région double brin.

10. Procédé de la revendication 1, dans lequel la molécule d'ARN exprimée comprend la séquence de répétition inversée SEQ ID N° 17 ou SEQ ID N° 18.

11. Procédé de la revendication 10, dans lequel l'expression de ladite molécule d'ARN est commandée par un promoteur hétérologue, spécifique de tissu, à régulation développementale, constitutif ou inductible.

12. Procédé de la revendication 11, dans lequel l'expression de ladite molécule d'ARN est commandée par un promoteur de l'ubiquitine, tel que le promoteur Ubi3 d'Arabidopsis.

13. Procédé de la revendication 8, dans lequel ladite molécule d'ADN comprend un lieur entre les séquences d'ADN codant pour ledit fragment d'ARN sens et lesdits fragments d'ARN antisens.

14. Procédé de la revendication 13, dans lequel ledit lieur comprend une cassette d'expression d'un gène fonctionnel, tel qu'un gène marqueur sélectionnable.

15. Procédé de la revendication 14, dans lequel ledit lieur comprend des séquences régulatrices telles que des signaux de maturation d'introns.

16. Procédé de la revendication 7, dans lequel ledit fragment d'ARN sens et ledit fragment d'ARN antisens sont présents dans deux molécules d'ARN distinctes.

17. Procédé de la revendication 16, dans lequel ladite première séquence d'ADN et ladite deuxième séquence d'ADN sont liées de manière opérationnelle par un promoteur bidirectionnel.

18. Procédé de la revendication 7, dans lequel ladite première séquence d'ADN et ladite deuxième séquence d'ADN sont présentes dans des brins complémentaires de ladite molécule d'ADN.

19. Procédé de la revendication 18, dans lequel ladite première séquence d'ADN est le brin d'ADN complémentaire de ladite deuxième séquence d'ADN de ladite molécule d'ADN.

20. Construction d'ADN altérant l'expression d'un génome viral ou d'une portion de ce dernier, comprenant une première séquence d'ADN capable d'exprimer dans une cellule un fragment d'ARN sens dudit génome viral et une deuxième séquence d'ADN capable d'exprimer dans la cellule un fragment d'ARN antisens dudit génome viral, dans laquelle ledit fragment d'ARN sens et ledit fragment d'ARN antisens sont capables de former une molécule d'ARN double brin, ladite cellule étant une cellule végétale qui est rendue résistante ou tolérante à un virus, ledit virus étant un furovirus, et lesdites séquences d'ADN comprenant un gène de la réplicase ou des portions de ce dernier.

21. Construction d'ADN de la revendication 20, ladite construction d'ADN comprenant un premier promoteur lié de manière opérationnelle à ladite première séquence d'ADN, et un deuxième promoteur lié de manière opérationnelle à ladite deuxième séquence d'ADN.

22. Construction d'ADN de la revendication 20, ladite construction d'ADN comprenant un promoteur bidirectionnel lié de manière opérationnelle à ladite première séquence d'ADN et à ladite deuxième séquence d'ADN.

23. Cellule présentant une expression altérée d'un gène cible viral comprenant une première séquence d'ADN capable d'exprimer dans ladite cellule un fragment d'ARN sens dudit gène cible viral et une deuxième séquence d'ADN capable d'exprimer dans ladite cellule un fragment d'ARN antisens dudit gène cible viral, dans laquelle ledit fragment d'ARN sens et ledit fragment d'ARN antisens sont capables de former une molécule d'ARN double brin, ladite cellule étant une cellule végétale qui est rendue résistante ou tolérante à un virus, ledit virus étant un furovirus, et ledit gène cible étant un gène de la réplicase ou une portion de ce dernier.

24. Plante et descendance de cette dernière, comprenant la cellule végétale de la revendication 23.

25. Plante de la revendication 24, ladite plante étant résistante ou tolérante à un virus.

26. Semences comprenant la cellule végétale de la revendication 23.


### SEQUENCE LISTING

<110> Novartis AG
<120> Regulation of Viral Gene Expression
<130> S-30959A
<140>
   <141>
<150> US 09/309038
   <151> 1999-05-10
<160> 28
<170> PatentIn Ver. 2.1
<210> 1
   <211> 27
   <212> DNA
   <213> Artificial Sequence
<220>
   <223> Description of Artificial Sequence: ds_Luc1
<400> 1
   cgcggatcct ggaagacgcc aaaaaca 27
<210> 2
   <211> 38
   <212> DNA
   <213> Artificial Sequence
<220>
   <223> Description of Artificial Sequence: ds_Luc2
<400> 2
   cggaagctta ggctcgccta atcgcagtat ccggaatg 38
<210> 3
   <211> 28
   <212> DNA
   <213> Artificial Sequence
<220>
   <223> Description of Artificial Sequence: ds_Luc3
<400> 3
   cggtctagag gaagacgcca aaaacata 28
<210> 4
   <211> 22
   <212> DNA
   <213> Artificial Sequence
<220>
   <223> Description of Artificial Sequence: JG-L
<400> 4
   gtacctcgag tctagactcg ag 22
<210> 5
   <211> 4732
   <212> DNA
   <213> Artificial Sequence
<220>
   <223> Description of Artificial Sequence: pVictorHINK
<400> 5
   gcacgaaccc cccgttcagc ccgaccgctg cgccttatcc ggtaactatc gtcttgagtc 60
   caacccggta agacacgact tatcgccact ggcagcagcc actggtaaca ggattagcag 120
   agcgaggtat gtaggcggtg ctacagagtt cttgaagtgg tggcctaact acggctacac 180
   tagaaggaca gtatttggta tctgcgctct gctgaagcca gttaccttcg gaaaaagagt 240
   tggtagctct tgatccggca aacaaaccac cgctggtagc ggtggttttt ttgtttgcaa 300
   gcagcagatt acgcgcagaa aaaaaggatc tcaagaagat cctttgatct tttctacggg 360
   gtctgacgct cagtggaacg aaaactcacg ttaagggatt ttggtcatga gattatcaaa 420
   aaggatcttc acctagatcc ttttgatccg gaattaattc ctgtggttgg catgcacata 480
   caaatggacg aacggataaa ccttttcacg cccttttaaa tatccgatta ttctaataaa 540
   cgctcttttc tcttaggttt acccgccaat atatcctgtc aaacactgat agtttaaact 600
   gaaggcggga aacgacaatc tgatcatgag cggagaatta agggagtcac gttatgaccc 660
   ccgccgatga cgcgggacaa gccgttttac gtttggaact gacagaaccg caacgctgca 720
   ggaattggcc gcagcggcca tttaaatcaa ttgggcgcgc cgaattcgag ctcggtaccc 780
   ggggatcctc tagagtcgac catggtgatc actgcaggca tgcaagcttc gtacgttaat 840
   taattcgaat ccggagcggc cgcacgcgtg ggcccgttta aacctcgaga gatctgctag 900
   ccctgcagga aatttaccgg tgcccgggcg gccagcatgg ccgtatccgc aatgtgttat 960
   taagttgtct aagcgtcaat ttgtttacac cacaatatat cctgccacca gccagccaac 1020
   agctccccga ccggcagctc ggcacaaaat caccactcga tacaggcagc ccatcagaat 1080
   taattctcat gtttgacagc ttatcatcga ctgcacggtg caccaatgct tctggcgtca 1140
   ggcagccatc ggaagctgtg gtatggctgt gcaggtcgta aatcactgca taattcgtgt 1200
   cgctcaaggc gcactcccgt tctggataat gttttttgcg ccgacatcat aacggttctg 1260
   gcaaatattc tgaaatgagc tgttgacaat taatcatcgg ctcgtataat gtgtggaatt 1320
   gtgagcggat aacaatttca cacaggaaac agaccatgag ggaagcggtg atcgccgaag 1380
   tatcgactca actatcagag gtagttggcg tcatcgagcg ccatctcgaa ccgacgttgc 1440
   tggccgtaca tttgtacggc tccgcagtgg atggcggcct gaagccacac agtgatattg 1500
   atttgctggt tacggtgacc gtaaggcttg atgaaacaac gcggcgagct ttgatcaacg 1560
   accttttgga aacttcggct tcccctggag agagcgagat tctccgcgct gtagaagtca 1620
   ccattgttgt gcacgacgac atcattccgt ggcgttatcc agctaagcgc gaactgcaat 1680
   ttggagaatg gcagcgcaat gacattcttg caggtatctt cgagccagcc acgatcgaca 1740
   ttgatctggc tatcttgctg acaaaagcaa gagaacatag cgttgccttg gtaggtccag 1800
   cggcggagga actctttgat ccggttcctg aacaggatct atttgaggcg ctaaatgaaa 1860
   ccttaacgct atggaactcg ccgcccgact gggctggcga tgagcgaaat gtagtgctta 1920
   cgttgtcccg catttggtac agcgcagtaa ccggcaaaat cgcgccgaag gatgtcgctg 1980
   ccgactgggc aatggagcgc ctgccggccc agtatcagcc cgtcatactt gaagctaggc 2040
   aggcttatct tggacaagaa gatcgcttgg cctcgcgcgc agatcagttg gaagaatttg 2100
   ttcactacgt gaaaggcgag atcaccaagg tagtcggcaa ataaagctct agtggatccc 2160
   cgaggaatcg gcgtgacggt cgcaaaccat ccggcccggt acaaatcggc gcggcgctgg 2220
   gtgatgacct ggtggagaag ttgaaggccg cgcaggccgc ccagcggcaa cgcatcgagg 2280
   cagaagcacg ccccggtgaa tcgtggcaag cggccgctga tcgaatccgc aaagaatccc 2340
   ggcaaccgcc ggcagccggt gcgccgtcga ttaggaagcc gcccaagggc gacgagcaac 2400
   cagatttttt cgttccgatg ctctatgacg tgggcacccg cgatagtcgc agcatcatgg 2460
   acgtggccgt tttccgtctg tcgaagcgtg accgacgagc tggcgaggtg atccgctacg 2520
   agcttccaga cgggcacgta gaggtttcag cagggccggc cggcatggcc agtgtgtggg 2580
   attacgacct ggtactgatg gcggtttccc atctaaccga atccatgaac cgataccggg 2640
   aagggaaggg agacaagccc ggccgcgtgt tccgtccaca cgttgcggac gtactcaagt 2700
   tctgccggcg agccgatggc ggaaagcaga aagacgacct ggtagaaacc tgcattcggt 2760
   taaacaccac gcacgttgcc atgcagcgta cgaagaaggc caagaacggc cgcctggtga 2820
   cggtatccga gggtgaagcc ttgattagcc gctacaagat cgtaaagagc gaaaccgggc 2880
   ggccggagta catcgagatc gagctagctg attggatgta ccgcgagatc acagaaggca 2940
   agaacccgga cgtgctgacg gttcaccccg attacttttt gatcgatccc ggcatcggcc 3000
   gttttctcta ccgcctggca cgccgcgccg caggcaaggc agaagccaga tggttgttca 3060
   agacgatcta cgaacgcagt ggcagcgccg gagagttcaa gaagttctgt ttcaccgtgc 3120
   gcaagctgat cgggtcaaat gacctgccgg agtacgattt gaaggaggag gcggggcagg 3180
   ctggcccgat cctagtcatg cgctaccgca acctgatcga gggcgaagca tccgccggtt 3240
   cctaatgtac ggagcagatg ctagggcaaa ttgccctagc aggggaaaaa ggtcgaaaag 3300
   gtctctttcc tgtggatagc acgtacattg ggaacccaaa gccgtacatt gggaaccgga 3360
   acccgtacat tgggaaccca aagccgtaca ttgggaaccg gtcacacatg taagtgactg 3420
   atataaaaga gaaaaaaggc gatttttccg cctaaaactc tttaaaactt attaaaactc 3480
   ttaaaacccg cctggcctgt gcataactgt ctggccagcg cacagccgaa gagctgcaaa 3540
   aagcgcctac ccttcggtcg ctgcgctccc tacgccccgc cgcttcgcgt cggcctatcg 3600
   cggccgctgg ccgctcaaaa atggctggcc tacggccagg caatctacca gggcgcggac 3660
   aagccgcgcc gtcgccactc gaccgccggc gctgaggtct gcctcgtgaa gaaggtgttg 3720
   ctgactcata ccaggcctga atcgccccat catccagcca gaaagtgagg gagccacggt 3780
   tgatgagagc tttgttgtag gtggaccagt tggtgatttt gaacttttgc tttgccacgg 3840
   aacggtctgc gttgtcggga agatgcgtga tctgatcctt caactcagca aaagttcgat 3900
   ttattcaaca aagccgccgt cccgtcaagt cagcgtaatg ctctgccagt gttacaacca 3960
   attaaccaat tctgattaga aaaactcatc gagcatcaaa tgaaactgca atttattcat 4020
   atcaggatta tcaataccat atttttgaaa aagccgtttc tgtaatgaag gagaaaactc 4080
   accgaggcag ttccatagga tggcaagatc ctggtatcgg tctgcgattc cgactcgtcc 4140
   aacatcaata caacctatta atttcccctc gtcaaaaata aggttatcaa gtgagaaatc 4200
   accatgagtg acgactgaat ccggtgagaa tggcaaaagc tctgcattaa tgaatcggcc 4260
   aacgcgcggg gagaggcggt ttgcgtattg ggcgctcttc cgcttcctcg ctcactgact 4320
   cgctgcgctc ggtcgttcgg ctgcggcgag cggtatcagc tcactcaaag gcggtaatac 4380
   ggttatccac agaatcaggg gataacgcag gaaagaacat gtgagcaaaa ggccagcaaa 4440
   aggccaggaa ccgtaaaaag gccgcgttgc tggcgttttt ccataggctc cgcccccctg 4500
   acgagcatca caaaaatcga cgctcaagtc agaggtggcg aaacccgaca ggactataaa 4560
   gataccaggc gtttccccct ggaagctccc tcgtgcgctc tcctgttccg accctgccgc 4620
   ttaccggata cctgtccgcc tttctccctt cgggaagcgt ggcgctttct caatgctcac 4680
   gctgtaggta tctcagttcg gtgtaggtcg ttcgctccaa gctgggctgt gt         4732
<210> 6
   <211> 24
   <212> DNA
   <213> Artificial Sequence
<220>
   <223> Description of Artificial Sequence: HiNK025bis
<400> 6
   caattaccat ggacacggtc gtgg 24
<210> 7
   <211> 30
   <212> DNA
   <213> Artificial Sequence
<220>
   <223> Description of Artificial Sequence: HiNK226
<400> 7
   gccaaatgtt tgaacgctgc agcctatttg 30
<210> 8
   <211> 24
   <212> DNA
   <213> Artificial Sequence
<220>
   <223> Description of Artificial Sequence: HiNK025bis2
<400> 8
   aatcgtccat ggatacggtc gtgg 24
<210> 9
   <211> 30
   <212> DNA
   <213> Artificial Sequence
<220>
   <223> Description of Artificial Sequence: HiNK226bis
<400> 9
   ctagggccgg gttcctctgc agcctatttg 30
<210> 10
   <211> 31
   <212> DNA
   <213> Artificial Sequence
<220>
   <223> Description of Artificial Sequence: HiNK251
<400> 10
   ctcccaggtt gagactgccc tgcagtgccc a 31
<210> 11
   <211> 26
   <212> DNA
   <213> Artificial Sequence
<220>
   <223> Description of Artificial Sequence: HiNK228
<400> 11
   ttaccatgca tacggtcgtg ggtagg 26
<210> 12
   <211> 26
   <212> DNA
   <213> Artificial Sequence
<220>
   <223> Description of Artificial Sequence: HiNK228bis
<400> 12
   cgttaatgca tacggtcgtg ggtagg 26
<210> 13
   <211> 3338
   <212> DNA
   <213> Artificial Sequence
<220>
   <223> Description of Artificial Sequence: BWYV duplex RNA gene
<220>
   <221> promoter
   <222> (1)..(1131)
   <223> RolC promoter
<220>
   <221> repeat_unit
   <222> (1132)..(1737)
   <223> BWYV CP inverted repeat fragment
<220>
   <221> stem_loop
   <222> (1738)..(2470)
   <223> 0.7 Kb spacer fragment downstream of BWYV CP gene
<220>
   <221> repeat_unit
   <222> (2471)..(3074)
   <223> BWYV CP inverted repeat fragment
<220>
   <221> terminator
   <222> (3076)..(3338)
   <223> NOS terminator
<400> 13
   ggatccggcg tcggaaactg gcgccaatca gacacagtct ctggtcggga aagccagagg 60
   tagtttggca acaatcacat caagatcgat gcgcaagaca cgggaggcct taaaatctgg 120
   atcaagcgaa aatactgcat gcgtgatcgt tcatgggttc atagtactgg gtttgctttt 180
   tcttgtcgtg ttgtttggcc ttagcgaaag gatgtcaaaa aaggatgccc ataattggga 240
   ggagtggggt aaagcttaaa gttggcccgc tattggattt cgcgaaagcg cattggcaaa 300
   cgtgaagatt gctgcattca agatactttt tctattttct ggttaagatg taaagtattg 360
   ccacaatcat attaattact aacattgtat atgtaatata gtgcggaaat tatctatgcc 420
   aaaatgatgt attaataata gcaataataa tatgtgttaa tctttttcaa tcgggaatac 480
   gtttaagcga ttatcgtgtt gaataaatta ttccaaaagg aaatacatgg ttttggagaa 540
   cctgctatag atatatgcca aatttacact agtttagtgg gtgcaaaact attatctctg 600
   tttctgagtt taataaaaaa taaataagca gggcgaatag cagttagcct aagaaggaat 660
   ggtggccatg tacgtgcttt taagagaccc tataataaat tgccagctgt gttgctttgg 720
   tgccgacagg cctaacgtgg ggtttagctt gacaaagtag cgcctttccg cagcataaat 780
   aaaggtaggc gggtgcgtcc cattattaaa ggaaaaagca aaagctgaga ttccatagac 840
   cacaaaccac cattattgga ggacagaacc tattccctca cgtgggtcgc tgagctttaa 900
   acctaataag taaaaacaat taaaagcagg caggtgtccc ttctatattc gcacaacgag 960
   gcgacgtgga gcatcgacag ccgcatccat taattaataa atttgtggac ctatacctaa 1020
   ctcaaatatt tttattattt gctccaatac gctaagagct ctggattata aatagtttgg 1080
   atgcttcgag ttatgggtac aagcaacctg tttcctactt tgttaccatg gacacggtcg 1140
   tgggtaggag aattatcaat ggaagaagac gaccacgcag gcaaacacga cgcgctcagc 1200
   gccctcagcc agtggttgtg gtccaaacct ctcgggcaac acaacgccga cctagacgac 1260
   gacgaagagg taacaaccgg acaggaagaa ctgttcctac cagaggagca ggttcgagcg 1320
   agacatttgt tttctcaaaa gacaatctcg cgggaagttc cagcggagca atcacgttcg 1380
   ggccgagtct atcagactgc ccggcattct ctaatggaat gctcaaggcc taccatgagt 1440
   ataaaatctc aatggtcatt ttggagttcg tctccgaagc ctcttcccaa aattccggtt 1500
   ccatcgctta cgagctggac ccacactgta aactcaactc cctttcctca actatcaaca 1560
   agttcgggat cacaaagccc gggaaaaggg cgtttacagc gtcttacatc aacggaacgg 1620
   aatggcacga cgttgccgag gaccaattca ggatcctcta caaaggcaat ggttcttcat 1680
   cgatagctgg ttctttcaga atcaccatta agtgtcaatt ccacaacccc aaataggctg 1740
   cagtgcccaa ctctctttgg tctttctgtc tttacggaac cggatgagcc ttgttcatca 1800
   agtgctatgg cgatcatccc tatctccatc atccgatctg tccaggctcc gtacgaaacg 1860
   gcgtaattat atttagattg cttctggaca gcgggtccaa caacaaagaa ggaggcattg 1920
   tcaccagtcg tcttgatatg acaagtcaaa tctccatctc tttccagaca ctgtccttga 1980
   ttgaaatgac agccattgag ttccatgtct gggtggccat acttcaaagt attatcggcc 2040
   ttgttgttgg ttatctccac attattgtaa atccccacgt tccaaccttc actaagatca 2100
   tcgttgtagg cgatgagacc atcttgtttg tccttgttag gatctgtggt gcttgaggtt 2160
   ggttgatacc cctccatcga tatttccacg gtccactcac cttgagggac tggcactatt 2220
   atcatcggga tggctttcaa agaattctgg gaataccatc gagaatcgag gttcgtccag 2280
   ttcatgttct cgtcctctat gtagcgaaac cgttgggacg gcatatcata caaagagatg 2340
   gcatcatccg tagattgagc cattatacga gtcacgggga ctccagtata gacgataaaa 2400
   cgatatttct tttggggtgt gggttgtgga gagggagaag gccctgggct agggccgggt 2460
   tcctcgtcta cctatttggg gttgtggaat tgacacttaa tggtgattct gaaagaacca 2520
   gctatcgatg aagaaccatt gcctttgtag aggatcctga attggtcctc ggcaacgtcg 2580
   tgccattccg ttccgttgat gtaagacgct gtaaacgccc ttttcccggg ctttgtgatc 2640
   ccgaacttgt tgatagttga ggaaagggag ttgagtttac agtgtgggtc cagctcgtaa 2700
   gcgatggaac cggaattttg ggaagaggct tcggagacga actccaaaat gaccattgag 2760
   attttatact catggtaggc cttgagcatt ccattagaga atgccgggca gtctgataga 2820
   ctcggcccga acgtgattgc tccgctggaa cttcccgcga gattgtcttt tgagaaaaca 2880
   aatgtctcgc tcgaacctgc tcctctggta ggaacagttc ttcctgtccg gttgttacct 2940
   cttcgtcgtc gtctaggtcg gcgttgtgtt gcccgagagg tttggaccac aaccactggc 3000
   tgagggcgct gagcgcgtcg tgtttgcctg cgtggtcgtc ttcttccatt gataattctc 3060
   ctacccacga ccgtatgcag atcgttcaaa catttggcaa taaagtttct taagattgaa 3120
   tcctgttgcc ggtcttgcga tgattatcat ataatttctg ttgaattacg ttaagcatgt 3180
   aataattaac atgtaatgca tgacgttatt tatgagatgg gtttttatga ttagagtccc 3240
   gcaattatac atttaatacg cgatagaaaa caaaatatag cgcgcaacct aggataaatt 3300
   atcgcgcgcg gtgtcatcta tgttactaga tctctaga 3338
<210> 14
   <211> 29
   <212> DNA
   <213> Artificial Sequence
<220>
   <223> Description of Artificial Sequence: HiNK285
<400> 14
   tcgtagaaga gaattcaccc aaactatcc 29
<210> 15
   <211> 29
   <212> DNA
   <213> Artificial Sequence
<220>
   <223> Description of Artificial Sequence: HiNK283
<400> 15
   aagaattgca ggatccacag gctcggtac 29
<210> 16
   <211> 24
   <212> DNA
   <213> Artificial Sequence
<220>
   <223> Description of Artificial Sequence: HiNK284
<400> 16
   ttccaacgaa ttcggtctca gaca 24
<210> 17
   <211> 3648
   <212> DNA
   <213> Artificial Sequence
<220>
   <223> Description of Artificial Sequence: pHiNK181
<220>
   <221> promoter
   <222> (1)..(1738)
   <223> ubiquitin 3 promoter plus intron
<220>
   <221> repeat_unit
   <222> (1739)..(2166)
   <223> BNYVV RNA1 repeat fragment
<220>
   <221> misc_feature
   <222> (2167)..(2946)
   <223> spacer fragment
<220>
   <221> repeat_unit
   <222> (2947)..(3375)
   <223> BNYVV RNA1 repeat fragment
<220>
   <221> sten_loop
   <222> (1739)..(3375)
   <223> Inverted repeat of BNYVV RNA1 fragment separated by spacer
<220>
   <221> terminator
   <222> (3376)..(3648)
   <223> NOS terminator
<400> 17
   ggtaccggat ttggagccaa gtctcataaa cgccattgtg gaagaaagtc ttgagttggt 60
   ggtaatgtaa cagagtagta agaacagaga agagagagag tgtgagatac atgaattgtc 120
   gggcaacaaa aatcctgaac atcttatttt agcaaagaga aagagttccg agtctgtagc 180
   agaagagtga ggagaaattt aagctcttgg acttgtgaat tgttccgcct cttgaatact 240
   tcttcaatcc tcatatattc ttcttctatg ttacctgaaa accggcattt aatctcgcgg 300
   gtttattccg gttcaacatt ttttttgttt tgagttatta tctgggctta ataacgcagg 360
   cctgaaataa attcaaggcc caactgtttt tttttttaag aagttgctgt taaaaaaaaa 420
   aaaagggaat taacaacaac aacaaaaaaa gataaagaaa ataataacaa ttactttaat 480
   tgtagactaa aaaaacatag attttatcat gaaaaaaaga gaaaagaaat aaaaacttgg 540
   atcaaaaaaa aaaacataca gatcttctaa ttattaactt ttcttaaaaa ttaggtcctt 600
   tttcccaaca attaggttta gagttttgga attaaaccaa aaagattgtt ctaaaaaata 660
   ctcaaatttg gtagataagt ttccttattt taattagtca atggtagata cttttttttc 720
   ttttctttat tagagtagat tagaatcttt tatgccaagt tttgataaat taaatcaaga 780
   agataaacta tcataatcaa catgaaatta aaagaaaaat ctcatatata gtattagtat 840
   tctctatata tattatgatt gcttattctt aatgggttgg gttaaccaag acatagtctt 900
   aatggaaaga atcttttttg aactttttcc ttattgatta aattcttcta tagaaaagaa 960
   agaaattatt tgaggaaaag tatatacaaa aagaaaaata gaaaaatgtc agtgaagcag 1020
   atgtaatgga tgacctaatc caaccaccac cataggatgt ttctacttga gtcggtcttt 1080
   taaaaacgca cggtggaaaa tatgacacgt atcatatgat tccttccttt agtttcgtga 1140
   taataatcct caactgatat cttccttttt ttgttttggc taaagatatt ttattctcat 1200
   taatagaaaa gacggttttg ggcttttggt ttgcgatata aagaagacct tcgtgtggaa 1260
   gataataatt catcctttcg tctttttctg actcttcaat ctctcccaaa gcctaaagcg 1320
   atctctgcaa atctctcgcg actctctctt tcaaggtata ttttctgatt ctttttgttt 1380
   ttgattcgta tctgatctcc aatttttgtt atgtggatta ttgaatcttt tgtataaatt 1440
   gcttttgaca atattgttcg tttcgtcaat ccagcttcta aattttgtcc tgattactaa 1500
   gatatcgatt cgtagtgttt acatctgtgt aatttcttgc ttgattgtga aattaggatt 1560
   ttcaaggacg atctattcaa tttttgtgtt ttctttgttc gattctctct gttttaggtt 1620
   tcttatgttt agatccgttt ctctttggtg ttgttttgat ttctcttacg gcttttgatt 1680
   tggtatatgt tcgctgattg gtttctactt gttctattgt tttatttcag gtggatccac 1740
   aggctcggta cttatttcga aaagtgagaa attctccatc atcgacacaa gatagtgttg 1800
   cacgtatggt tgctcagcta tttgtttctg attgtttggt gccaaatgta gctgatactt 1860
   tttctgcttc caatttgtgg cgaattatgg acaaagctat gcatgacatg gtcgcaaaaa 1920
   attaccaagg ccaaatggaa gaggagttta cgcgtaatgc taaactatat cgttttcagt 1980
   tgaaggatat tgaaaaacct ttgaaggacc cagagactga tttggcaaag gctggtcaag 2040
   ggatattggc atggtctaag gaggcacatg ttaagtttat ggttgctttt agagttttaa 2100
   atgatttgtt attgaagtca ttaaactcta atgttgttta cgataacaca atgtctgaga 2160
   ccgaattcac ccaaactatc ctcaacgggt ggcaatgaca taaaaacttc ccgtatcggg 2220
   aacatcatca taaattgctc acgcccgatt cgagacacag agataatagc atccaaccaa 2280
   cgttgaacat catcaacatt gcgacaagat aagctagcgt tacactccaa aaaatcagca 2340
   taaacagctg ggtctttggg aagattttta atccaatcac gcaaagattc ctggtattca 2400
   caaaaatgct tatactcacg gaacctgtgt gctgctatct tcgtcaattt acgcgaaaca 2460
   cttggaaaca aatgtccatt agataaagca taaccacaaa aagtgatagg aacatttaaa 2520
   tccaatttga aatccaagac agtttccttt ttaatcaact ttaacatttg atcgttaatt 2580
   ttcaaattag cctgcctttt aaaaccatca tcgcccttca tggccataca aaatggtccg 2640
   gtcccacgaa gcatagcatt taacatagca cccattaaaa tggtattacc aagcaaagtg 2700
   ccgggttctc cactagtctt aacataagac atatgtgctc tgacatatct ggactgcata 2760
   acatatttct cacgaaatga gaaataccaa tccaaaaaga agtcagaaat gcccaaagca 2820
   gcataaatat gtctttctat caattgggtg aaaacccctt gcccagaatc gcaagcagca 2880
   gcatcgataa ccccgtttat agcactatct ggtactgtat tcatggcggc atttattttt 2940
   ccaacaaatt cggtctcaga cattgtgtta tcgtaaacaa cattagagtt taatgacttc 3000
   aataacaaat catttaaaac tctaaaagca accataaact taacatgtgc ctccttagac 3060
   catgccaata tcccttgacc agcctttgcc aaatcagtct ctgggtcctt caaaggtttt 3120
   tcaatatcct tcaactgaaa acgatatagt ttagcattac gcgtaaactc ctcttccatt 3180
   tggccttggt aattttttgc gaccatgtca tgcatagctt tgtccataat tcgccacaaa 3240
   ttggaagcag aaaaagtatc agctacattt ggcaccaaac aatcagaaac aaatagctga 3300
   gcaaccatac gtgcaacact atcttgtgtc gatgatggag aatttctcac ttttcgaaat 3360
   aagtaccgag cctgtggatc ccccgaattt ccccgatcgt tcaaacattt ggcaataaag 3420
   tttcttaaga ttgaatcctg ttgccggtct tgcgatgatt atcatctaat ttctgttgaa 3480
   ttacgttaag catgtaataa ttaacatgta atgcatgacg ttatttatga gatgggtttt 3540
   tatgattaga gtcccgcaat tatacattta atacgcgata gaaaacaaaa tatagcgcgc 3600
   aaactaggat aaattatcgc gcgcggtgtc atctatgtta ctagatcc 3648
<210> 18
   <211> 3648
   <212> DNA
   <213> Artificial Sequence
<220>
   <223> Description of Artificial Sequence: pHiNK184
<220>
   <221> promoter
   <222> (1)..(1738)
   <223> ubiquitin 3 promoter plus leader intron
<220>
   <221> repeat_unit
   <222> (1739)..(2166)
   <223> BNYVV RNA1 repeat fragment
<220>
   <221> misc_feature
   <222> (2167)..(2946)
   <223> spacer
<220>
   <221> repeat_unit
   <222> complement (2947)..(3375))
   <223> BNYVV RNA1 repeat fragment
<220>
   <221> stem_loop
   <222> (1739)..(3375)
   <223> Inverted repeat of BNYVV RNA1 fragment separated by spacer
<220>
   <221> terminator
   <222> (3376)..(3648)
   <223> NOS terminator
<400> 18
   ggtaccggat ttggagccaa gtctcataaa cgccattgtg gaagaaagtc ttgagttggt 60
   ggtaatgtaa cagagtagta agaacagaga agagagagag tgtgagatac atgaattgtc 120
   gggcaacaaa aatcctgaac atcttatttt agcaaagaga aagagttccg agtctgtagc 180
   agaagagtga ggagaaattt aagctcttgg acttgtgaat tgttccgcct cttgaatact 240
   tcttcaatcc tcatatattc ttcttctatg ttacctgaaa accggcattt aatctcgcgg 300
   gtttattccg gttcaacatt ttttttgttt tgagttatta tctgggctta ataacgcagg 360
   cctgaaataa attcaaggcc caactgtttt tttttttaag aagttgctgt taaaaaaaaa 420
   aaaagggaat taacaacaac aacaaaaaaa gataaagaaa ataataacaa ttactttaat 480
   tgtagactaa aaaaacatag attttatcat gaaaaaaaga gaaaagaaat aaaaacttgg 540
   atcaaaaaaa aaaacataca gatcttctaa ttattaactt ttcttaaaaa ttaggtcctt 600
   tttcccaaca attaggttta gagttttgga attaaaccaa aaagattgtt ctaaaaaata 660
   ctcaaatttg gtagataagt ttccttattt taattagtca atggtagata cttttttttc 720
   ttttctttat tagagtagat tagaatcttt tatgccaagt tttgataaat taaatcaaga 780
   agataaacta tcataatcaa catgaaatta aaagaaaaat ctcatatata gtattagtat 840
   tctctatata tattatgatt gcttattctt aatgggttgg gttaaccaag acatagtctt 900
   aatggaaaga atcttttttg aactttttcc ttattgatta aattcttcta tagaaaagaa 960
   agaaattatt tgaggaaaag tatatacaaa aagaaaaata gaaaaatgtc agtgaagcag 1020
   atgtaatgga tgacctaatc caaccaccac cataggatgt ttctacttga gtcggtcttt 1080
   taaaaacgca cggtggaaaa tatgacacgt atcatatgat tccttccttt agtttcgtga 1140
   taataatcct caactgatat cttccttttt ttgttttggc taaagatatt ttattctcat 1200
   taatagaaaa gacggttttg ggcttttggt ttgcgatata aagaagacct tcgtgtggaa 1260
   gataataatt catcctttcg tctttttctg actcttcaat ctctcccaaa gcctaaagcg 1320
   atctctgcaa atctctcgcg actctctctt tcaaggtata ttttctgatt ctttttgttt 1380
   ttgattcgta tctgatctcc aatttttgtt atgtggatta ttgaatcttt tgtataaatt 1440
   gcttttgaca atattgttcg tttcgtcaat ccagcttcta aattttgtcc tgattactaa 1500
   gatatcgatt cgtagtgttt acatctgtgt aatttcttgc ttgattgtga aattaggatt 1560
   ttcaaggacg atctattcaa tttttgtgtt ttctttgttc gattctctct gttttaggtt 1620
   tcttatgttt agatccgttt ctctttggtg ttgttttgat ttctcttacg gcttttgatt 1680
   tggtatatgt tcgctgattg gtttctactt gttctattgt tttatttcag gtggatccac 1740
   aggctcggta cttatttcga aaagtgagaa attctccatc atcgacacaa gatagtgttg 1800
   cacgtatggt tgctcagcta tttgtttctg attgtttggt gccaaatgta gctgatactt 1860
   tttctgcttc caatttgtgg cgaattatgg acaaagctat gcatgacatg gtcgcaaaaa 1920
   attaccaagg ccaaatggaa gaggagttta cgcgtaatgc taaactatat cgttttcagt 1980
   tgaaggatat tgaaaaacct ttgaaggacc cagagactga tttggcaaag gctggtcaag 2040
   ggatattggc atggtctaag gaggcacatg ttaagtttat ggttgctttt agagttttaa 2100
   atgatttgtt attgaagtca ttaaactcta atgttgttta cgataacaca atgtctgaga 2160
   ccgaatttgt tggaaaaata aatgccgcca tgaatacagt accagatagt gctataaacg 2220
   gggttatcga tgctgctgct tgcgattctg ggcaaggggt tttcacccaa ttgatagaaa 2280
   gacatattta tgctgctttg ggcatttctg acttcttttt ggattggtat ttctcatttc 2340
   gtgagaaata tgttatgcag tccagatatg tcagagcaca tatgtcttat gttaagacta 2400
   gtggagaacc cggcactttg cttggtaata ccattttaat gggtgctatg ttaaatgcta 2460
   tgcttcgtgg gaccggacca ttttgtatgg ccatgaaggg cgatgatggt tttaaaaggc 2520
   aggctaattt gaaaattaac gatcaaatgt taaagttgat taaaaaggaa actgtcttgg 2580
   atttcaaatt ggatttaaat gttcctatca ctttttgtgg ttatgcttta tctaatggac 2640
   atttgtttcc aagtgtttcg cgtaaattga cgaagatagc agcacacagg ttccgtgagt 2700
   ataagcattt ttgtgaatac caggaatctt tgcgtgattg gattaaaaat cttcccaaag 2760
   acccagctgt ttatgctgat tttttggagt gtaacgctag cttatcttgt cgcaatgttg 2820
   atgatgttca acgttggttg gatgctatta tctctgtgtc tcgaatcggg cgtgagcaat 2880
   ttatgatgat gttcccgata cgggaagttt ttatgtcatt gccacccgtt gaggatagtt 2940
   tgggtgaatt cggtctcaga cattgtgtta tcgtaaacaa cattagagtt taatgacttc 3000
   aataacaaat catttaaaac tctaaaagca accataaact taacatgtgc ctccttagac 3060
   catgccaata tcccttgacc agcctttgcc aaatcagtct ctgggtcctt caaaggtttt 3120
   tcaatatcct tcaactgaaa acgatatagt ttagcattac gcgtaaactc ctcttccatt 3180
   tggccttggt aattttttgc gaccatgtca tgcatagctt tgtccataat tcgccacaaa 3240
   ttggaagcag aaaaagtatc agctacattt ggcaccaaac aatcagaaac aaatagctga 3300
   gcaaccatac gtgcaacact atcttgtgtc gatgatggag aatttctcac ttttcgaaat 3360
   aagtaccgag cctgtggatc ccccgaattt ccccgatcgt tcaaacattt ggcaataaag 3420
   tttcttaaga ttgaatcctg ttgccggtct tgcgatgatt atcatctaat ttctgttgaa 3480
   ttacgttaag catgtaataa ttaacatgta atgcatgacg ttatttatga gatgggtttt 3540
   tatgattaga gtcccgcaat tatacattta atacgcgata gaaaacaaaa tatagcgcgc 3600
   aaactaggat aaattatcgc gcgcggtgtc atctatgtta ctagatcc              3648
<210> 19
   <211> 41
   <212> DNA
   <213> Artificial Sequence
<220>
   <223> Description of Artificial Sequence: ZUP1563
<400> 19
   gggcggatcc gctagcccgc ggccgctctt tctttccaag g 41
<210> 20
   <211> 35
   <212> DNA
   <213> Artificial Sequence
<220>
   <223> Description of Artificial Sequence: ZUP1564
<400> 20
   cccgccatgg gtcgacgcca ttttttatga gctgc 35
<210> 21
   <211> 40
   <212> DNA
   <213> Artificial Sequence
<220>
   <223> Description of Artificial Sequence: ZUP1565
<400> 21
   cccgccatgg gatccgatga tttctaccga gaattaaggg 40
<210> 22
   <211> 29
   <212> DNA
   <213> Artificial Sequence
<220>
   <223> Description of Artificial Sequence: ZUP1566
<400> 22
   gggcgctagc ctaatgctta tatagtacc 29
<210> 23
   <211> 30
   <212> DNA
   <213> Artificial Sequence
<220>
   <223> Description of Artificial Sequence: ZUP1567
<400> 23
   gggcgctagc cttgctggag tataagccgg 30
<210> 24
   <211> 35
   <212> DNA
   <213> Artificial Sequence
<220>
   <223> Description of Artificial Sequence: ZUP1568
<400> 24
   gggcgtcgac cgcgggcttt aaaggtggga ggccc 35
<210> 25
   <211> 12766
   <212> DNA
   <213> Artificial Sequence
<220>
   <223> Description of Artificial Sequence: pZU623
<220>
   <221> promoter
   <222> (55)..(1781)
   <223> ubiquitin3 promoter plus leader intron
<220>
   <221> misc_feature
   <222> (1790)..(2430)
   <223> PRSV nt CP region
<220>
   <221> misc_feature
   <222> (2511)..(3111)
   <223> ZYMV nt CP region
<220>
   <221> intron
   <222> (3245)..(3686)
   <223> actin2 intronL
<220>
   <221> misc_feature
   <222> Complement ((3822)..(4422))
   <223> ZYMV nt CP region
<220>
   <221> misc_feature
   <222> Complement ((4503)..(5143))
   <223> PRSV nt CP region
<220>
   <221> terminator
   <222> (5160)..(5429)
   <223> NOS terminator
<220>
   <221> promoter
   <222> (5498)..(6669)
   <223> SMAS promoter
<220>
   <221> gene
   <222> (6691)..(7866)
   <223> phosphomannose isomerase A coding sequence
<220>
   <221> terminator
   <222> (7928)..(8202)
   <223> nopaline synthase terminator
<220>
   <221> misc_feature
   <222> (8254)..(8385)
   <223> nopaline left border fragment
<220>
   <221> gene
   <222> (8664)..(9452)
   <223> spectinomycin (aadA) coding sequence
<220>
   <221> misc_structure
   <222> (9462)..(11549)
   <223> pVS1 ORI
<220>
   <221> misc_structure
   <222> (11550)..(12484)
   <223> pUC19 ORI
<220>
   <221> misc_feature
   <222> (12500)..(12755)
   <223> nopaline right border region
<220>
   <221> stem_loop
   <222> (1790)..(5143)
   <223> PRSV-ZYMV inverted repeat fragment
<400> 25
   ggccgcagcg gccatttaaa tcaattgggc gcgccgaatt cgagctcggt accccggatt 60
   tggagccaag tctcataaac gccattgtgg aagaaagtct tgagttggtg gtaatgtaac 120
   agagtagtaa gaacagagaa gagagagagt gtgagataca tgaattgtcg ggcaacaaaa 180
   atcctgaaca tcttatttta gcaaagagaa agagttccga gtctgtagca gaagagtgag 240
   gagaaattta agctcttgga cttgtgaatt gttccgcctc ttgaatactt cttcaatcct 300
   catatattct tcttctatgt tacctgaaaa ccggcattta atctcgcggg tttattccgg 360
   ttcaacattt tttttgtttt gagttattat ctgggcttaa taacgcaggc ctgaaataaa 420
   ttcaaggccc aactgttttt tttttaagaa gttgctgtta aaaaaaaaaa aagggaatta 480
   acaacaacaa caaaaaaaga taaagaaaat aataacaatt actttaattg tagactaaaa 540
   aaacatagat tttatcatga aaaaaagaga aaagaaataa aaacttggat caaaaaaaaa 600
   aacatacaga tcttctaatt attaactttt cttaaaaatt aggtcctttt tcccaacaat 660
   taggtttaga gttttggaat taaaccaaaa agattgttct aaaaaatact caaatttggt 720
   agataagttt ccttatttta attagtcaat ggtagatact tttttttctt ttctttatta 780
   gagtagatta gaatctttta tgccaagttt tgataaatta aatcaagaag ataaactatc 840
   ataatcaaca tgaaattaaa agaaaaatct catatatagt attagtattc tctatatata 900
   ttatgattgc ttattcttaa tgggttgggt taaccaagac atagtcttaa tggaaagaat 960
   cttttttgaa ctttttcctt attgattaaa ttcttctata gaaaagaaag aaattatttg 1020
   aggaaaagta tatacaaaaa gaaaaataga aaaatgtcag tgaagcagat gtaatggatg 1080
   acctaatcca accaccacca taggatgttt ctacttgagt cggtctttta aaaacgcacg 1140
   gtggaaaata tgacacgtat catatgattc cttcctttag tttcgtgata ataatcctca 1200
   actgatatct tccttttttt gttttggcta aagatatttt attctcatta atagaaaaga 1260
   cggttttggg cttttggttt gcgatataaa gaagaccttc gtgtggaaga taataattca 1320
   tcctttcgtc tttttctgac tcttcaatct ctcccaaagc ctaaagcgat ctctgcaaat 1380
   ctctcgcgac tctctctttc aaggtatatt ttctgattct ttttgttttt gattcgtatc 1440
   tgatctccaa tttttgttat gtggattatt gaatcttttg tataaattgc ttttgacaat 1500
   attgttcgtt tcgtcaatcc agcttctaaa ttttgtcctg attactaaga tatcgattcg 1560
   tagtgtttac atctgtgtaa tttcttgctt gattgtgaaa ttaggatttt caaggacgat 1620
   ctattcaatt tttgtgtttt ctttgttcga ttctctctgt tttaggtttc ttatgtttag 1680
   atccgtttct ctttggtgtt gttttgattt ctcttacggc ttttgatttg gtatatgttc 1740
   gctgattggt ttctacttgt tctattgttt tatttcaggt gggggatccg atgatttcta 1800
   ccgagaatta agggaaagac tgtccttaat ttaaatcatc ttcttcagta taatccgcaa 1860
   caaattgaca tttctaacac tcgtgccact cagtcacaat ttgagaagtg gtatgaggga 1920
   gtgaggaatg attatggtct caatgataat gaaatgcaag tgatgctaaa tggcttgatg 1980
   gtttggtgta tcgagaatgg tacatctcca gacatatctg gtgtctgggt tatgatggat 2040
   ggggaaaccc aagttgatta tccaatcaag cctttaatag agcatgctac tccgtcattt 2100
   aggcaaatta tggctcactt tagtaacgcg gcagaagcat acattgcgaa gagaaatgct 2160
   actgagagat acatgccgcg gtatggaatc aagagaaatt tgactgacat tagtcttgct 2220
   agatacgctt tcgacttcta tgaggtgaat tcgaaaacac ctgatagggc tcgtgaagct 2280
   cacatgcaga tgaaagctgc agcgctgcga aacactagtc gcagaatgtt tggtatggac 2340
   ggcagtgtta gtaacaagga agaaaacacg gagagacaca cagtggaaga tgtcaataga 2400
   gacatgcact ctctcctggg tatgcgcaac tgaatactcg cgcttgtgtg tttgtcgagt 2460
   ctgactcgac cctgtttcac cttatggtac tatataagca ttaggctagc cttgctggag 2520
   tataagccgg atcaaattga gttatacaac acacgagcgt ctcatcagca attcgcctct 2580
   tggttcaacc aagttaaaac agaatatgat ctgaatgagc aacagatggg agttgtaatg 2640
   aatggtttca tggtttggtg cattgaaaat ggcacgtcac ccgacattaa cggagtatgg 2700
   gttatgatgg acggtaatga gcaggttgaa tatcctttga aaccaatagt tgaaaatgca 2760
   aagccaacgc tgcgacaaat aatgcatcac ttttcagatg cagcggaggc atatatagag 2820
   atgagaaatg cagaggcacc atacatgccg aggtatggtt tgcttcgaaa cttacgggat 2880
   aggagtttgg cacgatatgc tttcgacttc tacgaagtca attccaaaac tccggaaaga 2940
   gcccgcgaag ctgttgcgca gatgaaagca gcagccctta gcaatgtttc ttcaaggttg 3000
   tttggccttg atggaaatgt tgccaccact agcgaagaca ctgaacggca cactgcacgt 3060
   gatgttaata ggaacatgca caccttgcta ggtgtgaata caatgcagta aagggtaggt 3120
   cgcctaccta ggttatcgtt tcgctgccga cgtaattcta atatttaccg ctttatgtga 3180
   tgtctttaga tttctagagt gggcctccca cctttaaagc ccgcggccgc tctttctttc 3240
   caaggtaata ggaactttct ggatctactt tatttgctgg atctcgatct tgttttctca 3300
   atttccttga gatctggaat tcgtttaatt tggatctgtg aacctccact aaatcttttg 3360
   gttttactag aatcgatcta agttgaccga tcagttagct cgattatagc taccagaatt 3420
   tggcttgacc ttgatggaga gatccatgtt catgttacct gggaaatgat ttgtatatgt 3480
   gaattgaaat ctgaactgtt gaagttagat tgaatctgaa cactgtcaat gttagattga 3540
   atctgaacac tgtttaagtt agatgaagtt tgtgtataga ttcttcgaaa ctttaggatt 3600
   tgtagtgtcg tacgttgaac agaaagctat ttctgattca atcagggttt atttgactgt 3660
   attgaactct ttttgtgtgt ttgcagctca taaaaaatgg cgtcgaccgc gggctttaaa 3720
   ggtgggaggc ccactctaga aatctaaaga catcacataa agcggtaaat attagaatta 3780
   cgtcggcagc gaaacgataa cctaggtagg cgacctaccc tttactgcat tgtattcaca 3840
   cctagcaagg tgtgcatgtt cctattaaca tcacgtgcag tgtgccgttc agtgtcttcg 3900
   ctagtggtgg caacatttcc atcaaggcca aacaaccttg aagaaacatt gctaagggct 3960
   gctgctttca tctgcgcaac agcttcgcgg gctctttccg gagttttgga attgacttcg 4020
   tagaagtcga aagcatatcg tgccaaactc ctatcccgta agtttcgaag caaaccatac 4080
   ctcggcatgt atggtgcctc tgcatttctc atctctatat atgcctccgc tgcatctgaa 4140
   aagtgatgca ttatttgtcg cagcgttggc tttgcatttt caactattgg tttcaaagga 4200
   tattcaacct gctcattacc gtccatcata acccatactc cgttaatgtc gggtgacgtg 4260
   ccattttcaa tgcaccaaac catgaaacca ttcattacaa ctcccatctg ttgctcattc 4320
   agatcatatt ctgttttaac ttggttgaac caagaggcga attgctgatg agacgctcgt 4380
   gtgttgtata actcaatttg atccggctta tactccagca aggctagcct aatgcttata 4440
   tagtaccata aggtgaaaca gggtcgagtc agactcgaca aacacacaag cgcgagtatt 4500
   cagttgcgca tacccaggag agagtgcatg tctctattga catcttccac tgtgtgtctc 4560
   tccgtgtttt cttccttgtt actaacactg ccgtccatac caaacattct gcgactagtg 4620
   tttcgcagcg ctgcagcttt catctgcatg tgagcttcac gagccctatc aggtgttttc 4680
   gaattcacct catagaagtc gaaagcgtat ctagcaagac taatgtcagt caaatttctc 4740
   ttgattccat accgcggcat gtatctctca gtagcatttc tcttcgcaat gtatgcttct 4800
   gccgcgttac taaagtgagc cataatttgc ctaaatgacg gagtagcatg ctctattaaa 4860
   ggcttgattg gataatcaac ttgggtttcc ccatccatca taacccagac accagatatg 4920
   tctggagatg taccattctc gatacaccaa accatcaagc catttagcat cacttgcatt 4980
   tcattatcat tgagaccata atcattcctc actccctcat accacttctc aaattgtgac 5040
   tgagtggcac gagtgttaga aatgtcaatt tgttgcggat tatactgaag aagatgattt 5100
   aaattaagga cagtctttcc cttaattctc ggtagaaatc atcggatcca tggtgatcac 5160
   tgcagatcgt tcaaacattt ggcaataaag tttcttaaga ttgaatcctg ttgccggtct 5220
   tgcgatgatt atcatataat ttctgttgaa ttacgttaag catgtaataa ttaacatgta 5280
   atgcatgacg ttatttatga gatgggtttt tatgattaga gtcccgcaat tatacattta 5340
   atacgcgata gaaaacaaaa tatagcgcgc aaactaggat aaattatcgc gcgcggtgtc 5400
   atctatgtta ctagatctct agaaagcttc gtacgttaat taattcgaat ccggagcggc 5460
   cgcagggcta gcatcgatgg taccgagctc gagactatac aggccaaatt cgctcttagc 5520
   cgtacaatat tactcaccgg tgcgatgccc cccatcgtag gtgaaggtgg aaattaatga 5580
   tccatcttga gaccacaggc ccacaacagc taccagtttc ctcaagggtc caccaaaaac 5640
   gtaagcgctt acgtacatgg tcgataagaa aaggcaattt gtagatgtta acatccaacg 5700
   tcgctttcag ggatcccgaa ttccaagctt ggaattcggg atcctacagg ccaaattcgc 5760
   tcttagccgt acaatattac tcaccggtgc gatgcccccc atcgtaggtg aaggtggaaa 5820
   ttaatgatcc atcttgagac cacaggccca caacagctac cagtttcctc aagggtccac 5880
   caaaaacgta agcgcttacg tacatggtcg ataagaaaag gcaatttgta gatgttaaca 5940
   tccaacgtcg ctttcaggga tcccgaattc caagcttgga attcgggatc ctacaggcca 6000
   aattcgctct tagccgtaca atattactca ccggtgcgat ccccccatcg taggtgaagg 6060
   tggaaattaa tgatccatct tgagaccaca ggcccacaac agctaccagt ttcctcaagg 6120
   gtccaccaaa aacgtaagcg cttacgtaca tggtcgataa gaaaaggcaa tttgtagatg 6180
   ttaacatcca acgtcgcttt cagggatccc gaattccaag cttgggctgc aggtcaatcc 6240
   cattgctttt gaagcagctc aacattgatc tctttctcga gggagatttt tcaaatcagt 6300
   gcgcaagacg tgacgtaagt atccgagtca gtttttattt ttctactaat ttggtcgttt 6360
   atttcggcgt gtaggacatg gcaaccgggc ctgaatttcg cgggtattct gtttctattc 6420
   caactttttc ttgatccgca gccattaacg acttttgaat agatacgctg acacgccaag 6480
   cctcgctagt caaaagtgta ccaaacaacg ctttacagca agaacggaat gcgcgtgacg 6540
   ctcgcggtga cgccatttcg ccttttcaga aatggataaa tagccttgct tcctattata 6600
   tcttcccaaa ttaccaatac attacactag catctgaatt tcataaccaa tctcgataca 6660
   ccaaatcgag atctgcaggg atccccgatc atgcaaaaac tcattaactc agtgcaaaac 6720
   tatgcctggg gcagcaaaac ggcgttgact gaactttatg gtatggaaaa tccgtccagc 6780
   cagccgatgg ccgagctgtg gatgggcgca catccgaaaa gcagttcacg agtgcagaat 6840
   gccgccggag atatcgtttc actgcgtgat gtgattgaga gtgataaatc gactctgctc 6900
   ggagaggccg ttgccaaacg ctttggcgaa ctgcctttcc tgttcaaagt attatgcgca 6960
   gcacagccac tctccattca ggttcatcca aacaaacaca attctgaaat cggttttgcc 7020
   aaagaaaatg ccgcaggtat cccgatggat gccgccgagc gtaactataa agatcctaac 7080
   cacaagccgg agctggtttt tgcgctgacg cctttccttg cgatgaacgc gtttcgtgaa 7140
   ttttccgaga ttgtctccct actccagccg gtcgcaggtg cacatccggc gattgctcac 7200
   tttttacaac agcctgatgc cgaacgttta agcgaactgt tcgccagcct gttgaatatg 7260
   cagggtgaag aaaaatcccg cgcgctggcg attttaaaat cggccctcga tagccagcag 7320
   ggtgaaccgt ggcaaacgat tcgtttaatt tctgaatttt acccggaaga cagcggtctg 7380
   ttctccccgc tattgctgaa tgtggtgaaa ttgaaccctg gcgaagcgat gttcctgttc 7440
   gctgaaacac cgcacgctta cctgcaaggc gtggcgctgg aagtgatggc aaactccgat 7500
   aacgtgctgc gtgcgggtct gacgcctaaa tacattgata ttccggaact ggttgccaat 7560
   gtgaaattcg aagccaaacc ggctaaccag ttgttgaccc agccggtgaa acaaggtgca 7620
   gaactggact tcccgattcc agtggatgat tttgccttct cgctgcatga ccttagtgat 7680
   aaagaaacca ccattagcca gcagagtgcc gccattttgt tctgcgtcga aggcgatgca 7740
   acgttgtgga aaggttctca gcagttacag cttaaaccgg gtgaatcagc gtttattgcc 7800
   gccaacgaat caccggtgac tgtcaaaggc cacggccgtt tagcgcgtgt ttacaacaag 7860
   ctgtaagagc ttactgaaaa aattaacatc tcttgctaag ctgggagctc gtcgacggat 7920
   cgaattcctg cagatcgttc aaacatttgg caataaagtt tcttaagatt gaatcctgtt 7980
   gccggtcttg cgatgattat catataattt ctgttgaatt acgttaagca tgtaataatt 8040
   aacatgtaat gcatgacgtt atttatgaga tgggttttta tgattagagt cccgcaatta 8100
   tacatttaat acgcgataga aaacaaaata tagcgcgcaa actaggataa attatcgcgc 8160
   gcggtgtcat ctatgttact agatctctag aactagtgga tctgctagcc ctgcaggaaa 8220
   tttaccggtg cccgggcggc cagcatggcc gtatccgcaa tgtgttatta agttgtctaa 8280
   gcgtcaattt gtttacacca caatatatcc tgccaccagc cagccaacag ctccccgacc 8340
   ggcagctcgg cacaaaatca ccactcgata caggcagccc atcagaatta attctcatgt 8400
   ttgacagctt atcatcgact gcacggtgca ccaatgcttc tggcgtcagg cagccatcgg 8460
   aagctgtggt atggctgtgc aggtcgtaaa tcactgcata attcgtgtcg ctcaaggcgc 8520
   actcccgttc tggataatgt tttttgcgcc gacatcataa cggttctggc aaatattctg 8580
   aaatgagctg ttgacaatta atcatcggct cgtataatgt gtggaattgt gagcggataa 8640
   caatttcaca caggaaacag accatgaggg aagcggtgat cgccgaagta tcgactcaac 8700
   tatcagaggt agttggcgtc atcgagcgcc atctcgaacc gacgttgctg gccgtacatt 8760
   tgtacggctc cgcagtggat ggcggcctga agccacacag tgatattgat ttgctggtta 8820
   cggtgaccgt aaggcttgat gaaacaacgc ggcgagcttt gatcaacgac cttttggaaa 8880
   cttcggcttc ccctggagag agcgagattc tccgcgctgt agaagtcacc attgttgtgc 8940
   acgacgacat cattccgtgg cgttatccag ctaagcgcga actgcaattt ggagaatggc 9000
   agcgcaatga cattcttgca ggtatcttcg agccagccac gatcgacatt gatctggcta 9060
   tcttgctgac aaaagcaaga gaacatagcg ttgccttggt aggtccagcg gcggaggaac 9120
   tctttgatcc ggttcctgaa caggatctat ttgaggcgct aaatgaaacc ttaacgctat 9180
   ggaactcgcc gcccgactgg gctggcgatg agcgaaatgt agtgcttacg ttgtcccgca 9240
   tttggtacag cgcagtaacc ggcaaaatcg cgccgaagga tgtcgctgcc gactgggcaa 9300
   tggagcgcct gccggcccag tatcagcccg tcatacttga agctaggcag gcttatcttg 9360
   gacaagaaga tcgcttggcc tcgcgcgcag atcagttgga agaatttgtt cactacgtga 9420
   aaggcgagat caccaaggta gtcggcaaat aaagctctag tggatccccg aggaatcggc 9480
   gtgacggtcg caaaccatcc ggcccggtac aaatcggcgc ggcgctgggt gatgacctgg 9540
   tggagaagtt gaaggccgcg caggccgccc agcggcaacg catcgaggca gaagcacgcc 9600
   ccggtgaatc gtggcaagcg gccgctgatc gaatccgcaa agaatcccgg caaccgccgg 9660
   cagccggtgc gccgtcgatt aggaagccgc ccaagggcga cgagcaacca gattttttcg 9720
   ttccgatgct ctatgacgtg ggcacccgcg atagtcgcag catcatggac gtggccgttt 9780
   tccgtctgtc gaagcgtgac cgacgagctg gcgaggtgat ccgctacgag cttccagacg 9840
   ggcacgtaga ggtttcagca gggccggccg gcatggccag tgtgtgggat tacgacctgg 9900
   tactgatggc ggtttcccat ctaaccgaat ccatgaaccg ataccgggaa gggaagggag 9960
   acaagcccgg ccgcgtgttc cgtccacacg ttgcggacgt actcaagttc tgccggcgag 10020
   ccgatggcgg aaagcagaaa gacgacctgg tagaaacctg cattcggtta aacaccacgc 10080
   acgttgccat gcagcgtacg aagaaggcca agaacggccg cctggtgacg gtatccgagg 10140
   gtgaagcctt gattagccgc tacaagatcg taaagagcga aaccgggcgg ccggagtaca 10200
   tcgagatcga gctagctgat tggatgtacc gcgagatcac agaaggcaag aacccggacg 10260
   tgctgacggt tcaccccgat tactttttga tcgatcccgg catcggccgt tttctctacc 10320
   gcctggcacg ccgcgccgca ggcaaggcag aagccagatg gttgttcaag acgatctacg 10380
   aacgcagtgg cagcgccgga gagttcaaga agttctgttt caccgtgcgc aagctgatcg 10440
   ggtcaaatga cctgccggag tacgatttga aggaggaggc ggggcaggct ggcccgatcc 10500
   tagtcatgcg ctaccgcaac ctgatcgagg gcgaagcatc cgccggttcc taatgtacgg 10560
   agcagatgct agggcaaatt gccctagcag gggaaaaagg tcgaaaaggt ctctttcctg 10620
   tggatagcac gtacattggg aacccaaagc cgtacattgg gaaccggaac ccgtacattg 10680
   ggaacccaaa gccgtacatt gggaaccggt cacacatgta agtgactgat ataaaagaga 10740
   aaaaaggcga tttttccgcc taaaactctt taaaacttat taaaactctt aaaacccgcc 10800
   tggcctgtgc ataactgtct ggccagcgca cagccgaaga gctgcaaaaa gcgcctaccc 10860
   ttcggtcgct gcgctcccta cgccccgccg cttcgcgtcg gcctatcgcg gccgctggcc 10920
   gctcaaaaat ggctggccta cggccaggca atctaccagg gcgcggacaa gccgcgccgt 10980
   cgccactcga ccgccggcgc tgaggtctgc ctcgtgaaga aggtgttgct gactcatacc 11040
   aggcctgaat cgccccatca tccagccaga aagtgaggga gccacggttg atgagagctt 11100
   tgttgtaggt ggaccagttg gtgattttga acttttgctt tgccacggaa cggtctgcgt 11160
   tgtcgggaag atgcgtgatc tgatccttca actcagcaaa agttcgattt attcaacaaa 11220
   gccgccgtcc cgtcaagtca gcgtaatgct ctgccagtgt tacaaccaat taaccaattc 11280
   tgattagaaa aactcatcga gcatcaaatg aaactgcaat ttattcatat caggattatc 11340
   aataccatat ttttgaaaaa gccgtttctg taatgaagga gaaaactcac cgaggcagtt 11400
   ccataggatg gcaagatcct ggtatcggtc tgcgattccg actcgtccaa catcaataca 11460
   acctattaat ttcccctcgt caaaaataag gttatcaagt gagaaatcac catgagtgac 11520
   gactgaatcc ggtgagaatg gcaaaagctc tgcattaatg aatcggccaa cgcgcgggga 11580
   gaggcggttt gcgtattggg cgctcttccg cttcctcgct cactgactcg ctgcgctcgg 11640
   tcgttcggct gcggcgagcg gtatcagctc actcaaaggc ggtaatacgg ttatccacag 11700
   aatcagggga taacgcagga aagaacatgt gagcaaaagg ccagcaaaag gccaggaacc 11760
   gtaaaaaggc cgcgttgctg gcgtttttcc ataggctccg cccccctgac gagcatcaca 11820
   aaaatcgacg ctcaagtcag aggtggcgaa acccgacagg actataaaga taccaggcgt 11880
   ttccccctgg aagctccctc gtgcgctctc ctgttccgac cctgccgctt accggatacc 11940
   tgtccgcctt tctcccttcg ggaagcgtgg cgctttctca atgctcacgc tgtaggtatc 12000
   tcagttcggt gtaggtcgtt cgctccaagc tgggctgtgt gcacgaaccc cccgttcagc 12060
   ccgaccgctg cgccttatcc ggtaactatc gtcttgagtc caacccggta agacacgact 12120
   tatcgccact ggcagcagcc actggtaaca ggattagcag agcgaggtat gtaggcggtg 12180
   ctacagagtt cttgaagtgg tggcctaact acggctacac tagaaggaca gtatttggta 12240
   tctgcgctct gctgaagcca gttaccttcg gaaaaagagt tggtagctct tgatccggca 12300
   aacaaaccac cgctggtagc ggtggttttt ttgtttgcaa gcagcagatt acgcgcagaa 12360
   aaaaaggatc tcaagaagat cctttgatct tttctacggg gtctgacgct cagtggaacg 12420
   aaaactcacg ttaagggatt ttggtcatga gattatcaaa aaggatcttc acctagatcc 12480
   ttttgatccg gaattaattc ctgtggttgg catgcacata caaatggacg aacggataaa 12540
   ccttttcacg cccttttaaa tatccgatta ttctaataaa cgctcttttc tcttaggttt 12600
   acccgccaat atatcctgtc aaacactgat agtttaaact gaaggcggga aacgacaatc 12660
   tgatcatgag cggagaatta agggagtcac gttatgaccc ccgccgatga cgcgggacaa 12720
   gccgttttac gtttggaact gacagaaccg caacgctgca ggaatt                12766
<210> 26
   <211> 42
   <212> DNA
   <213> Artificial Sequence
<220>
   <223> Description of Artificial Sequence: ZUP1598
<400> 26
   catgccatgg atccaatggc cacgaattaa agctatcacg tc 42
<210> 27
   <211> 44
   <212> DNA
   <213> Artificial Sequence
<220>
   <223> Description of Artificial Sequence: ZUP1590
<400> 27
   acgcgtcgac cgcggattca aacgattatt aattacgata aaag 44
<210> 28
   <211> 11461
   <212> DNA
   <213> Artificial Sequence
<220>
   <223> Description of Artificial Sequence: pZU634
<220>
   <221> promoter
   <222> (55)..(1786)
   <223> ubiquitin3 promoter plus leader intron
<220>
   <221> misc_feature
   <222> (1790)..(2574)
   <223> PVY nt CP region
<220>
   <221> intron
   <222> (2595)..(3036)
   <223> actin2 intronL
<220>
   <221> misc_feature
   <222> Complement ((3057)..(3847))
   <223> PVY nt CP region
<220>
   <221> terminator
   <222> (3855)..(4124)
   <223> NOS terminator
<220>
   <221> promoter
   <222> (4193)..(5364)
   <223> SMAS promoter
<220>
   <221> gene
   <222> (5386)..(6561)
   <223> phosphomannose isomerase A coding sequence
<220>
   <221> terminator
   <222> (6623)..(6897)
   <223> nopaline synthase terminator
<220>
   <221> misc_feature
   <222> (6949)..(7080)
   <223> nopaline left border fragment
<220>
   <221> gene
   <222> (7359)..(8147)
   <223> spectinomycin (aadA) coding sequence
<220>
   <221> misc_structure
   <222> (8157)..(10244)
   <223> pVS1 ORI
<220>
   <221> misc_structure
   <222> (10245)..(11179)
   <223> pUC19 ORI
<220>
   <221> misc_feature
   <222> (11195)..(11450)
   <223> nopaline right border region
<220>
   <221> stem_loop
   <222> (1790)..(3847)
   <223> PVY inverted repeat region
<400> 28
   ggccgcagcg gccatttaaa tcaattgggc gcgccgaatt cgagctcggt acccgtaccg 60
   gatttggagc caagtctcat aaacgccatt gtggaagaaa gtcttgagtt ggtggtaatg 120
   taacagagta gtaagaacag agaagagaga gagtgtgaga tacatgaatt gtcgggcaac 180
   aaaaatcctg aacatcttat tttagcaaag agaaagagtt ccgagtctgt agcagaagag 240
   tgaggagaaa tttaagctct tggacttgtg aattgttccg cctcttgaat acttcttcaa 300
   tcctcatata ttcttcttct atgttacctg aaaaccggca tttaatctcg cgggtttatt 360
   ccggttcaac attttttttg ttttgagtta ttatctgggc ttaataacgc aggcctgaaa 420
   taaattcaag gcccaactgt tttttttttt aagaagttgc tgttaaaaaa aaaaaaaggg 480
   aattaacaac aacaacaaaa aaagataaag aaaataataa caattacttt aattgtagac 540
   taaaaaaaca tagattttat catgaaaaaa agagaaaaga aataaaaact tggatcaaaa 600
   aaaaaaacat acagatcttc taattattaa cttttcttaa aaattaggtc ctttttccca 660
   acaattaggt ttagagtttt ggaattaaac caaaaagatt gttctaaaaa atactcaaat 720
   ttggtagata agtttcctta ttttaattag tcaatggtag atactttttt ttcttttctt 780
   tattagagta gattagaatc ttttatgcca agttttgata aattaaatca agaagataaa 840
   ctatcataat caacatgaaa ttaaaagaaa aatctcatat atagtattag tattctctat 900
   atatattatg attgcttatt cttaatgggt tgggttaacc aagacatagt cttaatggaa 960
   agaatctttt ttgaactttt tccttattga ttaaattctt ctatagaaaa gaaagaaatt 1020
   atttgaggaa aagtatatac aaaaagaaaa atagaaaaat gtcagtgaag cagatgtaat 1080
   ggatgaccta atccaaccac caccatagga tgtttctact tgagtcggtc ttttaaaaac 1140
   gcacggtgga aaatatgaca cgtatcatat gattccttcc tttagtttcg tgataataat 1200
   cctcaactga tatcttcctt tttttgtttt ggctaaagat attttattct cattaataga 1260
   aaagacggtt ttgggctttt ggtttgcgat ataaagaaga ccttcgtgtg gaagataata 1320
   attcatcctt tcgtcttttt ctgactcttc aatctctccc aaagcctaaa gcgatctctg 1380
   caaatctctc gcgactctct ctttcaaggt atattttctg attctttttg tttttgattc 1440
   gtatctgatc tccaattttt gttatgtgga ttattgaatc ttttgtataa attgcttttg 1500
   acaatattgt tcgtttcgtc aatccagctt ctaaattttg tcctgattac taagatatcg 1560
   attcgtagtg tttacatctg tgtaatttct tgcttgattg tgaaattagg attttcaagg 1620
   acgatctatt caatttttgt gttttctttg ttcgattctc tctgttttag gtttcttatg 1680
   tttagatccg tttctctttg gtgttgtttt gatttctctt acggcttttg atttggtata 1740
   tgttcgctga ttggtttcta cttgttctat tgttttattt caggtggggg atccaatggc 1800
   cacgaattaa agctatcacg tccaaaatga gaatgcccaa gagtaagggt gcaactgtac 1860
   taaatttgga acacctactc gagtatgctc cacagcaaat tgaaatctca aatactcgag 1920
   caactcaatc acagtttgat acatggtatg aagcagtaca acttgcatac gacataggag 1980
   aaactgaaat gccaactgtg atgaatgggc ttatggtttg gtgcattgaa aatggaacct 2040
   cgccaaatat caatggagtt tgggttatga tggatggaga tgaacaagtc gaatacccac 2100
   tgaaaccaat cgttgagaat gcaaaaccaa cacttaggca aatcatggca catttctcag 2160
   atgttgcaga agcgtatata gaaatgcgca acaaaaagga accatatatg ccacgatatg 2220
   gtttagttcg taatctgcgc gatggaagtt tggctcgcta tgcttttgac ttttatgaag 2280
   ttacatcacg tacaccagtg agggctagag aggcacacat tcaaatgaag gccgcagctt 2340
   taaaatcagc tcaatctcga cttttcggat tggatggtgg cattagtaca caagaggaaa 2400
   acacagagag gcacaccacc gaggatgttt ctccaagtat gcatactcta cttggagtga 2460
   agaacatgtg attgtagtgt ctttccggac gatatataga tatttatgtt tgcagtaagt 2520
   attttggctt ttcctgtact acttttatcg taattaataa tcgtttgaat ccgcggccgc 2580
   tctttctttc caaggtaata ggaactttct ggatctactt tatttgctgg atctcgatct 2640
   tgttttctca atttccttga gatctggaat tcgtttaatt tggatctgtg aacctccact 2700
   aaatcttttg gttttactag aatcgatcta agttgaccga tcagttagct cgattatagc 2760
   taccagaatt tggcttgacc ttgatggaga gatccatgtt catgttacct gggaaatgat 2820
   ttgtatatgt gaattgaaat ctgaactgtt gaagttagat tgaatctgaa cactgtcaat 2880
   gttagattga atctgaacac tgtttaagtt agatgaagtt tgtgtataga ttcttcgaaa 2940
   ctttaggatt tgtagtgtcg tacgttgaac agaaagctat ttctgattca atcagggttt 3000
   atttgactgt attgaactct ttttgtgtgt ttgcagctca taaaaaatgg cgtcgaccgc 3060
   ggattcaaac gattattaat tacgataaaa gtagtacagg aaaagccaaa atacttactg 3120
   caaacataaa tatctatata tcgtccggaa agacactaca atcacatgtt cttcactcca 3180
   agtagagtat gcatacttgg agaaacatcc tcggtggtgt gcctctctgt gttttcctct 3240
   tgtgtactaa tgccaccatc caatccgaaa agtcgagatt gagctgattt taaagctgcg 3300
   gccttcattt gaatgtgtgc ctctctagcc ctcactggtg tacgtgatgt aacttcataa 3360
   aagtcaaaag catagcgagc caaacttcca tcgcgcagat tacgaactaa accatatcgt 3420
   ggcatatatg gttccttttt gttgcgcatt tctatatacg cttctgcaac atctgagaaa 3480
   tgtgccatga tttgcctaag tgttggtttt gcattctcaa cgattggttt cagtgggtat 3540
   tcgacttgtt catctccatc catcataacc caaactccat tgatatttgg cgaggttcca 3600
   ttttcaatgc accaaaccat aagcccattc atcacagttg gcatttcagt ttctcctatg 3660
   tcgtatgcaa gttgtactgc ttcataccat gtatcaaact gtgattgagt tgctcgagta 3720
   tttgagattt caatttgctg tggagcatac tcgagtaggt gttccaaatt tagtacagtt 3780
   gcacccttac tcttgggcat tctcattttg gacgtgatag ctttaattcg tggccattgg 3840
   atccatggtg atcactgcag atcgttcaaa catttggcaa taaagtttct taagattgaa 3900
   tcctgttgcc ggtcttgcga tgattatcat ataatttctg ttgaattacg ttaagcatgt 3960
   aataattaac atgtaatgca tgacgttatt tatgagatgg gtttttatga ttagagtccc 4020
   gcaattatac atttaatacg cgatagaaaa caaaatatag cgcgcaacct aggataaatt 4080
   atcgcgcgcg gtgtcatcta tgttactaga tctctagaaa gcttcgtacg ttaattaatt 4140
   cgaatccgga gcggccgcag ggctagcatc gatggtaccg agctcgagac tatacaggcc 4200
   aaattcgctc ttagccgtac aatattactc accggtgcga tgccccccat cgtaggtgaa 4260
   ggtggaaatt aatgatccat cttgagacca caggcccaca acagctacca gtttcctcaa 4320
   gggtccacca aaaacgtaag cgcttacgta catggtcgat aagaaaaggc aatttgtaga 4380
   tgttaacatc caacgtcgct ttcagggatc ccgaattcca agcttggaat tcgggatcct 4440
   acaggccaaa ttcgctctta gccgtacaat attactcacc ggtgcgatgc cccccatcgt 4500
   aggtgaaggt ggaaattaat gatccatctt gagaccacag gcccacaaca gctaccagtt 4560
   tcctcaaggg tccaccaaaa acgtaagcgc ttacgtacat ggtcgataag aaaaggcaat 4620
   ttgtagatgt taacatccaa cgtcgctttc agggatcccg aattccaagc ttggaattcg 4680
   ggatcctaca ggccaaattc gctcttagcc gtacaatatt actcaccggt gcgatccccc 4740
   catcgtaggt gaaggtggaa attaatgatc catcttgaga ccacaggccc acaacagcta 4800
   ccagtttcct caagggtcca ccaaaaacgt aagcgcttac gtacatggtc gataagaaaa 4860
   ggcaatttgt agatgttaac atccaacgtc gctttcaggg atcccgaatt ccaagcttgg 4920
   gctgcaggtc aatcccattg cttttgaagc agctcaacat tgatctcttt ctcgagggag 4980
   atttttcaaa tcagtgcgca agacgtgacg taagtatccg agtcagtttt tatttttcta 5040
   ctaatttggt cgtttatttc ggcgtgtagg acatggcaac cgggcctgaa tttcgcgggt 5100
   attctgtttc tattccaact ttttcttgat ccgcagccat taacgacttt tgaatagata 5160
   cgctgacacg ccaagcctcg ctagtcaaaa gtgtaccaaa caacgcttta cagcaagaac 5220
   ggaatgcgcg tgacgctcgc ggtgacgcca tttcgccttt tcagaaatgg ataaatagcc 5280
   ttgcttccta ttatatcttc ccaaattacc aatacattac actagcatct gaatttcata 5340
   accaatctcg atacaccaaa tcgagatctg cagggatccc cgatcatgca aaaactcatt 5400
   aactcagtgc aaaactatgc ctggggcagc aaaacggcgt tgactgaact ttatggtatg 5460
   gaaaatccgt ccagccagcc gatggccgag ctgtggatgg gcgcacatcc gaaaagcagt 5520
   tcacgagtgc agaatgccgc cggagatatc gtttcactgc gtgatgtgat tgagagtgat 5580
   aaatcgactc tgctcggaga ggccgttgcc aaacgctttg gcgaactgcc tttcctgttc 5640
   aaagtattat gcgcagcaca gccactctcc attcaggttc atccaaacaa acacaattct 5700
   gaaatcggtt ttgccaaaga aaatgccgca ggtatcccga tggatgccgc cgagcgtaac 5760
   tataaagatc ctaaccacaa gccggagctg gtttttgcgc tgacgccttt ccttgcgatg 5820
   aacgcgtttc gtgaattttc cgagattgtc tccctactcc agccggtcgc aggtgcacat 5880
   ccggcgattg ctcacttttt acaacagcct gatgccgaac gtttaagcga actgttcgcc 5940
   agcctgttga atatgcaggg tgaagaaaaa tcccgcgcgc tggcgatttt aaaatcggcc 6000
   ctcgatagcc agcagggtga accgtggcaa acgattcgtt taatttctga attttacccg 6060
   gaagacagcg gtctgttctc cccgctattg ctgaatgtgg tgaaattgaa ccctggcgaa 6120
   gcgatgttcc tgttcgctga aacaccgcac gcttacctgc aaggcgtggc gctggaagtg 6180
   atggcaaact ccgataacgt gctgcgtgcg ggtctgacgc ctaaatacat tgatattccg 6240
   gaactggttg ccaatgtgaa attcgaagcc aaaccggcta accagttgtt gacccagccg 6300
   gtgaaacaag gtgcagaact ggacttcccg attccagtgg atgattttgc cttctcgctg 6360
   catgacctta gtgataaaga aaccaccatt agccagcaga gtgccgccat tttgttctgc 6420
   gtcgaaggcg atgcaacgtt gtggaaaggt tctcagcagt tacagcttaa accgggtgaa 6480
   tcagcgttta ttgccgccaa cgaatcaccg gtgactgtca aaggccacgg ccgtttagcg 6540
   cgtgtttaca acaagctgta agagcttact gaaaaaatta acatctcttg ctaagctggg 6600
   agctcgtcga cggatcgaat tcctgcagat cgttcaaaca tttggcaata aagtttctta 6660
   agattgaatc ctgttgccgg tcttgcgatg attatcatat aatttctgtt gaattacgtt 6720
   aagcatgtaa taattaacat gtaatgcatg acgttattta tgagatgggt ttttatgatt 6780
   agagtcccgc aattatacat ttaatacgcg atagaaaaca aaatatagcg cgcaaactag 6840
   gataaattat cgcgcgcggt gtcatctatg ttactagatc tctagaacta gtggatctgc 6900
   tagccctgca ggaaatttac cggtgcccgg gcggccagca tggccgtatc cgcaatgtgt 6960
   tattaagttg tctaagcgtc aatttgttta caccacaata tatcctgcca ccagccagcc 7020
   aacagctccc cgaccggcag ctcggcacaa aatcaccact cgatacaggc agcccatcag 7080
   aattaattct catgtttgac agcttatcat cgactgcacg gtgcaccaat gcttctggcg 7140
   tcaggcagcc atcggaagct gtggtatggc tgtgcaggtc gtaaatcact gcataattcg 7200
   tgtcgctcaa ggcgcactcc cgttctggat aatgtttttt gcgccgacat cataacggtt 7260
   ctggcaaata ttctgaaatg agctgttgac aattaatcat cggctcgtat aatgtgtgga 7320
   attgtgagcg gataacaatt tcacacagga aacagaccat gagggaagcg gtgatcgccg 7380
   aagtatcgac tcaactatca gaggtagttg gcgtcatcga gcgccatctc gaaccgacgt 7440
   tgctggccgt acatttgtac ggctccgcag tggatggcgg cctgaagcca cacagtgata 7500
   ttgatttgct ggttacggtg accgtaaggc ttgatgaaac aacgcggcga gctttgatca 7560
   acgacctttt ggaaacttcg gcttcccctg gagagagcga gattctccgc gctgtagaag 7620
   tcaccattgt tgtgcacgac gacatcattc cgtggcgtta tccagctaag cgcgaactgc 7680
   aatttggaga atggcagcgc aatgacattc ttgcaggtat cttcgagcca gccacgatcg 7740
   acattgatct ggctatcttg ctgacaaaag caagagaaca tagcgttgcc ttggtaggtc 7800
   cagcggcgga ggaactcttt gatccggttc ctgaacagga tctatttgag gcgctaaatg 7860
   aaaccttaac gctatggaac tcgccgcccg actgggctgg cgatgagcga aatgtagtgc 7920
   ttacgttgtc ccgcatttgg tacagcgcag taaccggcaa aatcgcgccg aaggatgtcg 7980
   ctgccgactg ggcaatggag cgcctgccgg cccagtatca gcccgtcata cttgaagcta 8040
   ggcaggctta tcttggacaa gaagatcgct tggcctcgcg cgcagatcag ttggaagaat 8100
   ttgttcacta cgtgaaaggc gagatcacca aggtagtcgg caaataaagc tctagtggat 8160
   ccccgaggaa tcggcgtgac ggtcgcaaac catccggccc ggtacaaatc ggcgcggcgc 8220
   tgggtgatga cctggtggag aagttgaagg ccgcgcaggc cgcccagcgg caacgcatcg 8280
   aggcagaagc acgccccggt gaatcgtggc aagcggccgc tgatcgaatc cgcaaagaat 8340
   cccggcaacc gccggcagcc ggtgcgccgt cgattaggaa gccgcccaag ggcgacgagc 8400
   aaccagattt tttcgttccg atgctctatg acgtgggcac ccgcgatagt cgcagcatca 8460
   tggacgtggc cgttttccgt ctgtcgaagc gtgaccgacg agctggcgag gtgatccgct 8520
   acgagcttcc agacgggcac gtagaggttt cagcagggcc ggccggcatg gccagtgtgt 8580
   gggattacga cctggtactg atggcggttt cccatctaac cgaatccatg aaccgatacc 8640
   gggaagggaa gggagacaag cccggccgcg tgttccgtcc acacgttgcg gacgtactca 8700
   agttctgccg gcgagccgat ggcggaaagc agaaagacga cctggtagaa acctgcattc 8760
   ggttaaacac cacgcacgtt gccatgcagc gtacgaagaa ggccaagaac ggccgcctgg 8820
   tgacggtatc cgagggtgaa gccttgatta gccgctacaa gatcgtaaag agcgaaaccg 8880
   ggcggccgga gtacatcgag atcgagctag ctgattggat gtaccgcgag atcacagaag 8940
   gcaagaaccc ggacgtgctg acggttcacc ccgattactt tttgatcgat cccggcatcg 9000
   gccgttttct ctaccgcctg gcacgccgcg ccgcaggcaa ggcagaagcc agatggttgt 9060
   tcaagacgat ctacgaacgc agtggcagcg ccggagagtt caagaagttc tgtttcaccg 9120
   tgcgcaagct gatcgggtca aatgacctgc cggagtacga tttgaaggag gaggcggggc 9180
   aggctggccc gatcctagtc atgcgctacc gcaacctgat cgagggcgaa gcatccgccg 9240
   gttcctaatg tacggagcag atgctagggc aaattgccct agcaggggaa aaaggtcgaa 9300
   aaggtctctt tcctgtggat agcacgtaca ttgggaaccc aaagccgtac attgggaacc 9360
   ggaacccgta cattgggaac ccaaagccgt acattgggaa ccggtcacac atgtaagtga 9420
   ctgatataaa agagaaaaaa ggcgattttt ccgcctaaaa ctctttaaaa cttattaaaa 9480
   ctcttaaaac ccgcctggcc tgtgcataac tgtctggcca gcgcacagcc gaagagctgc 9540
   aaaaagcgcc tacccttcgg tcgctgcgct ccctacgccc cgccgcttcg cgtcggccta 9600
   tcgcggccgc tggccgctca aaaatggctg gcctacggcc aggcaatcta ccagggcgcg 9660
   gacaagccgc gccgtcgcca ctcgaccgcc ggcgctgagg tctgcctcgt gaagaaggtg 9720
   ttgctgactc ataccaggcc tgaatcgccc catcatccag ccagaaagtg agggagccac 9780
   ggttgatgag agctttgttg taggtggacc agttggtgat tttgaacttt tgctttgcca 9840
   cggaacggtc tgcgttgtcg ggaagatgcg tgatctgatc cttcaactca gcaaaagttc 9900
   gatttattca acaaagccgc cgtcccgtca agtcagcgta atgctctgcc agtgttacaa 9960
   ccaattaacc aattctgatt agaaaaactc atcgagcatc aaatgaaact gcaatttatt 10020
   catatcagga ttatcaatac catatttttg aaaaagccgt ttctgtaatg aaggagaaaa 10080
   ctcaccgagg cagttccata ggatggcaag atcctggtat cggtctgcga ttccgactcg 10140
   tccaacatca atacaaccta ttaatttccc ctcgtcaaaa ataaggttat caagtgagaa 10200
   atcaccatga gtgacgactg aatccggtga gaatggcaaa agctctgcat taatgaatcg 10260
   gccaacgcgc ggggagaggc ggtttgcgta ttgggcgctc ttccgcttcc tcgctcactg 10320
   actcgctgcg ctcggtcgtt cggctgcggc gagcggtatc agctcactca aaggcggtaa 10380
   tacggttatc cacagaatca ggggataacg caggaaagaa catgtgagca aaaggccagc 10440
   aaaaggccag gaaccgtaaa aaggccgcgt tgctggcgtt tttccatagg ctccgccccc 10500
   ctgacgagca tcacaaaaat cgacgctcaa gtcagaggtg gcgaaacccg acaggactat 10560
   aaagatacca ggcgtttccc cctggaagct ccctcgtgcg ctctcctgtt ccgaccctgc 10620
   cgcttaccgg atacctgtcc gcctttctcc cttcgggaag cgtggcgctt tctcaatgct 10680
   cacgctgtag gtatctcagt tcggtgtagg tcgttcgctc caagctgggc tgtgtgcacg 10740
   aaccccccgt tcagcccgac cgctgcgcct tatccggtaa ctatcgtctt gagtccaacc 10800
   cggtaagaca cgacttatcg ccactggcag cagccactgg taacaggatt agcagagcga 10860
   ggtatgtagg cggtgctaca gagttcttga agtggtggcc taactacggc tacactagaa 10920
   ggacagtatt tggtatctgc gctctgctga agccagttac cttcggaaaa agagttggta 10980
   gctcttgatc cggcaaacaa accaccgctg gtagcggtgg tttttttgtt tgcaagcagc 11040
   agattacgcg cagaaaaaaa ggatctcaag aagatccttt gatcttttct acggggtctg 11100
   acgctcagtg gaacgaaaac tcacgttaag ggattttggt catgagatta tcaaaaagga 11160
   tcttcaccta gatccttttg atccggaatt aattcctgtg gttggcatgc acatacaaat 11220
   ggacgaacgg ataaaccttt tcacgccctt ttaaatatcc gattattcta ataaacgctc 11280
   ttttctctta ggtttacccg ccaatatatc ctgtcaaaca ctgatagttt aaactgaagg 11340
   cgggaaacga caatctgatc atgagcggag aattaaggga gtcacgttat gacccccgcc 11400
   gatgacgcgg gacaagccgt tttacgtttg gaactgacag aaccgcaacg ctgcaggaat 11460
   t                                                                 11461